(19) Europäisches Patentamt

European Patent Office

Office européen des brevets

(11) **EP 1 019 715 B1**

(12) **EUROPEAN PATENT SPECIFICATION**

(45) Date of publication and mention
of the grant of the patent:
**26.01.2005 Bulletin 2005/04**

(21) Application number: **98939894.6**

(22) Date of filing: **07.08.1998**

(51) Int Cl.7: **G01N 33/00**, G01N 27/12,
G01N 27/22

(86) International application number:
**PCT/US1998/016527**

(87) International publication number:
**WO 1999/008105 (18.02.1999 Gazette 1999/07)**

(54) **TECHNIQUES AND SYSTEMS FOR ANALYTE DETECTION**

TECHNIKEN UND SYSTEME ZUM NACHWEIS VON ANALYTEN

TECHNIQUES ET SYSTEMES DE DETECTION D'ANALYSATS

(84) Designated Contracting States:
**AT BE CH CY DE DK ES FI FR GB GR IE IT LI LU
MC NL PT SE**
Designated Extension States:
**AL LT LV MK RO SI**

(30) Priority: **08.08.1997 US 55071 P
09.04.1998 US 81182 P**

(43) Date of publication of application:
**19.07.2000 Bulletin 2000/29**

(73) Proprietors:
• **CALIFORNIA INSTITUTE OF TECHNOLOGY
Pasadena, California 91125 (US)**
• **Cyrano Sciences Inc.
Pasadena, CA 91107 (US)**

(72) Inventors:
• **GOODMAN, Rodney, M.
Altadena, CA 91001 (US)**
• **LEWIS, Nathan, S.
La Canada, CA 91011 (US)**
• **GRUBBS, Robert, H.
South Pasadena, CA 91030 (US)**
• **DIXSON, Jeffrey
Pasadena, CA 91106 (US)**

(74) Representative: **Bizley, Richard Edward et al
Hepworth, Lawrence, Bryer & Bizley
Merlin House
Falconry Court
Baker's Lane
Epping Essex CM16 5DQ (GB)**

(56) References cited:
EP-A- 0 774 662          WO-A-93/22678
WO-A-96/30750          US-A- 5 345 213
US-A- 5 571 401

• **PERSAUD K C ET AL: "DESIGN STRATEGIES
FOR GAS AND ODOUR SENSORS WHICH MIMIC
THE OLFACTORY SYSTEM" NATO ASI SERIES.
SERIES F: COMPUTER AND SYSTEM
SCIENCES, no. 102, 1993, pages 579-602,
XP000645746**

**Description**

The research carried out in this application was supported in part by grants from the United States Army (#DAAG55-97-1-0187), DARPA (DAAK60-97-K-9503), and the National Science Foundation (CHE 9202583). The U. S. government may have rights in any patent issuing from this application.

The field of the invention relates generally to sensor arrays and techniques for the detection of analytes, and more specifically, though not exclusively, to electronic techniques and devices for olfaction.

Human beings have at least five senses: sight, smell, taste, hearing, and touch. Since the earliest times, humankind has sought techniques and devices for enhancing and extending these senses. Many of the devices and instruments that have been developed to extend human perception are considered among of the most revolutionary inventions in history. These inventions have had a profound impact on human civilization and have led to many additional breakthroughs and discoveries. Just a few of the many instruments developed to extend the reach of human perception include the telescope, microscope, stethoscope, X-rays, phonograph/radio/audio amplifier, scanning electron microscope, night vision goggles, and many, many others.

As would be expected, there has been considerable interest in developing a device or instrument for the general detection of analytes in a fluid, vacuum, air, or other medium.

More particularly, there is considerable interest in developing sensors that act as analogs of the mammalian olfactory system (Lundstrom *et al*. (1991) Nature 352:47-50; Shurmer and Gardner (1992) *Sens. Act. B. 8*:1-11). This system is thought to utilize probabilistic repertoires of many different receptors to recognize a single odorant (Reed (1992) *Neuron 8*:205-209; Lancet and Ben-Airie (1993) *Curr. Biol. 3*:668-674). In such a configuration, the burden of recognition is not on highly specific receptors, as in the traditional "lock-and-key" molecular recognition approach to chemical sensing, but lies instead on the distributed pattern processing of the olfactory bulb and the brain (Kauer (1991) *TINS 14*:79-85; DeVries and Baylor (1993) *Cell 10*(S) : 139-149).

Prior attempts to produce a broadly responsive sensor array have exploited heated metal oxide thin film resistors (Gardner *et al*. (1991) *Sens. Act. B 4*:117-121; Gardner *et al*. (1991) *Sens. Act. B 6*:71-75; Corcoran *et al*. (1993) *Sens. Act. B* 15:32-37), polymer sorption layers on the surfaces of acoustic wave resonators (Grate and Abraham (1991) *Sens. Act. B 3*:85-111; Grate *et al*. (1993) *Anal. Chem. 65*:1868-1881), arrays of electrochemical detectors (Stetter *et al*. (1986) *Anal. Chem. 58*:860-866; Stetter *et al*. (1990) *Sens. Act. B 1*:43-47; Stetter *et al*. (1993) *Anal. Chem. Acta 284*:1-11), or conductive polymers (Pearce *et al*. (1993) *Analyst 118*:371-377; Shurmer et al. (1991)*Sens. Act. B 4*:29-33; Lewis, *et al*. US Patent No. 5,571,401). Arrays of metal oxide thin film resistors, typically based on $SnO_2$ films that have been coated with various catalysts, yield distinct, diagnostic responses for several vapors (Gardner *et al*. (1991) *Sens. Act. B 4*:117-121; Gardner *et al*. (1991) *Sens. Act. B 6*:71-75; Corcoran *et al*. (1993) *Sens. Act. B 15*:32-37). However, due to the lack of understanding of catalyst function, $SnO_2$ arrays do not allow deliberate chemical control of the response of elements in the arrays nor reproducibility of response from array to array. Surface acoustic wave resonators are extremely sensitive to both mass and acoustic impedance changes of the coatings in array elements, but the signal transduction mechanism involves somewhat complicated electronics, requiring frequency measurement to 1 Hz while sustaining a 100 MHz Rayleigh wave in the crystal (Grate and Abraham (1991) *Sens. Act. B 3*: 85-111; Grate *et al*. (1993) *Anal. Chem. 65*:1868-1881). Attempts have been made to construct sensors with conducting polymer elements that have been grown electrochemically through nominally identical polymer films and coatings (Pearce *et al*. (1993) *Analyst 118*:371-377; Shurmer *et al*. (1991) *Sens. Act. B 4*:29-33; Topart and Josowicz (1992) *J. Phys. Chem. 96*:7824-7830; Charlesworth *et al*. (1993) *J. Phys. Chem. 97*:5418-5423). Also dielectric films and capacitors have been used.

Pearce *et al*. (1993) *Analyst 118*:371-377 and Gardner et al. (1994) *Sensors and Actuators B 18-19*:240-243 describe polypyrrole-based sensor arrays for monitoring beer flavor. Shurmer (1990) US Patent No. 4,907,441, describes general sensor arrays with particular electrical circuitry. See also Gabor Harasa'nyi, POLYMER FILMS IN SENSOR APPLICATIONS (1995), Technomic Publishing Co., Lancaster, PA.

A specific instance of an analyte detector is a device for sensing smell or odors (i.e., analytes in air). It is well recognized that some animals like dogs have a keener sense of smell than human beings. Because of their "noses", dogs have been utilized for many tasks including, for example, the detection of bombs, mines, drugs, poison gases, and illegal contraband; dogs also aid in the search and rescue of humans. Devices for sensing smell would be useful for the traditional applications where animals are used, as well as for a multitude of uses where animals are impractical or inappropriate.

Moreover, a device for the general detection of analytes has potentially many more applications than a specific device for detecting smells. For example, the uses for a device for analyte detection include the detection of chemical leaks, quality control in food processing, medical diagnosis and testing, fabrication and manufacture of commercial and industrial goods, pharmaceutical production, testing or evaluating any odorant or analyte in any medium (e.g., fuel, oil, wine, solvents), and many other applications. An instrument for analyte detection would be highly desirable in industries and applications such as the chemical and petrochemical sectors, food, fragrance, medical, automotive, military, environmental, health and safety, and indoor air quality. Therefore, it is desirable to develop techniques and devices for the detection of analytes.

An approach for sensing smells is to use surface acoustic wave (SAW) resonators. However, the signal transduction mechanism for SAW devices involves relatively complicated electronics, and are thus somewhat costly. Furthermore, SAW devices are generally extremely sensitive to both mass and acoustic impedance changes, and may not be suitable for use in all environments.

Therefore, there is a need for techniques and systems for analyte detection, especially ones that are low cost, easy to manufacture, provide rapid response, and produce accurate differentiation between different analytes and different concentrations of the same analyte.

SUMMARY OF THE INVENTION

Accordingly, it is an object of the present invention to provide an integrated circuit and an electronic system for detecting analytes in fluids.

The invention provides an integrated circuit comprising a plurality of sensor sites formed on the integrated circuit, wherein a sensor material is constrained at the sensor site and the sensor material has regions of a nonconductive organic material and a conductive material, and, in the presence of an analyte, the sensor material having measurable changes in an electrical property; and electrical terminals formed to couple to the sensor material at the sensor sites, wherein the electrical terminals transmit electrical signals to evaluate the change in the electrical property of the sensor material; characterized by the sensor site comprising a sensor hurdle.

Analytes may include smells, tastes, vapors, odors, gases, liquids, and chemicals, among others. The fluid may be liquid or gaseous in nature. In one embodiment of the present invention, an analyte is sensed by sensors that output electrical signals in response to the analyte. The electrical signals may be preprocessed by filtering and amplification. This preprocessing may also include adapting the sensor and electronics to the environment in which the analyte exists. The electrical signals may be further processed to classify and identify the analyte.

There are many possible embodiments of an analyte detection system of the present invention. For example, the present invention may be used to implement an electronic olfaction system or "electronic nose". Such a system may reveal the identification and concentration of vapors in a manner similar to the mammalian olfactory system. Another embodiment for the analyte detection system of the present invention may also be used to implement a device for tasting. This device would function similarly to a tongue. There are many other possible embodiments of the present invention, too numerous to name in this application.

The sensor material applied to or formed at one sensor site may have a different composition from the sensor material at a different site. For example, each sensor in the analyte detection system may have a different composition from every other sensor. For example, the sensor material may consist of regions of a nonconductive organic insulating material and a conductive material such as carbon black; the composition of carbon black may vary for each sensor on the chip. By providing a system of diverse sensors, each sensor may have a different response characteristic for a given analyte.

The integrated circuit may also include an electrical connection at each sensor site to route the electrical signals from the sensor material to other circuitry. This circuitry may further process the electrical signals. The circuitry may be on the same chip (on-chip) with the sensors, or may be off the chip carrying the sensors (off-chip), such as on a different integrated circuit. For example, an analyte detection system of the present invention may include two or more integrated circuits, making up an analyte detection chipset.

In a specific embodiment of the present invention, electronic circuitry resides on the same integrated circuit as the sensor site. In particular, there is a circuitry associated with each sensor site, and this circuitry may be formed beneath or interspersed with the sensor sites.

The signals from the sensors may be further processed by classifying the response to the analyte. For example, each analyte may have a particular "fingerprint". The analyte may be identified based on this fingerprint. The signal processing for the identification and classification of the analyte may be performed by on-chip or off-chip circuitry. For example, classification may be performed using a computer or other instrument, among other techniques. Therefore, using the techniques and system of the present invention, an analyte may be distinguished and identified.

The integrated circuit of the invention can be used in an electronic system for olfaction.

In specific embodiments, the electrical property may be a resistance, capacitance, inductance, or other electrical property. The sensor material may be a material consisting of a nonconductive organic insulating material and a conductive material.

In a further embodiment, the integrated circuit of the present invention includes an array of sensors for detecting chemical analytes, each sensor having a first and second output terminal. There may be a plurality of adaptive electronic circuits, each circuit associated with one of the sensors and coupled to the first and second output terminals of the associated sensor.

Other objects, features, and advantages of the preent invention will become apparent upon consideration of the following detailed description and the accompanying drawings, in which like reference designations represent like features throughout the figures.

BRIEF DESCRIPTION OF THE DRAWINGS

Figure 1 shows a substrate with a number of analyte detection integrated circuits;

Figure 2A shows a more detailed diagram of one analyte detection integrated circuit, incorporating, particularly, sensor wells, which are not of the present invention;

Figure 2B shows a detailed view of a sensor well (not of the present invention);

Figure 2C shows an embodiment of the present invention in which a detection chip is formed with only a single conducting layer;

Figure 3 shows how a sensor array including a collection of different sensors may be used to identify an analyte;

Figure 4 shows a cross section of a sensor well (not of the present invention);

Figure 5 shows a top view of a layout of a sensor well (not of the present invention);

Figure 6 shows a layout of an integrated circuit with a number of sensor wells (not of the present invention);

Figure 7 shows a top view of a layout for a sensor site, where electronic circuitry is formed beneath the sensor site;

Figures 8A through 8F show the different stages in a process of fabricating a sensor site and depositing the sensor material (not of the present invention);

Figure 9 shows a cross section of an embodiment of a sensor site formed by planarizing an insulator layer (not of the present invention);

Figure 10 shows a cross section of another embodiment of a sensor site (according to the present invention);

Figure 11 shows a cross section of a further embodiment of a sensor site (not of the present invention);

Figure 12 shows an equivalent circuit diagram for the case of a discontinuous film on top of a continuous high-impedance film;

Figure 13 is a block diagram of a technique for evaluating or measuring the capacitance of a sensor to detect an analyte;

Figure 14 shows another embodiment for evaluating or measuring the capacitance of a sensor element;

Figure 15 shows a layout of capacitive sensor sites for an integrated circuit;

Figure 16 shows a unit cell;

Figure 17 shows a diagram of circuitry for reading out data from an array of sensors;

Figure 18 shows a diagram of an analyte detection system;

Figure 19 shows a specific embodiment of an analyte detection system;

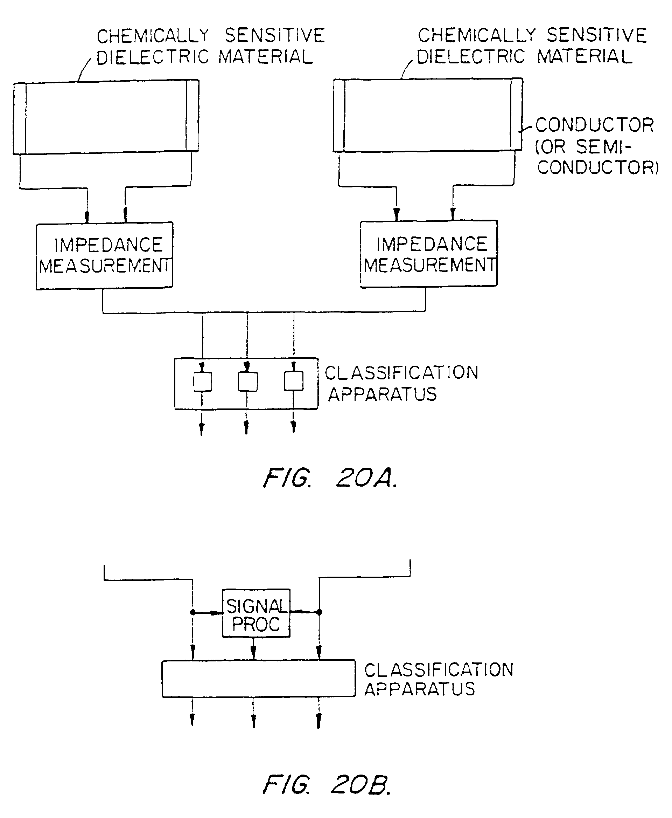

FIG. 20A is a simplified block diagram of a fluid identification apparatus that measures the capacitive effect a fluid has on two distinct, chemically sensitive dielectric materials and that then classifies the resulting capacitance measurements, to determine the identity of the fluid;

FIG. 20B is a simplified block diagram of an alternative configuration for the classification portion of the apparatus of FIG. 20A, this alternative configuration including preliminary signal processing of the capacitance measurements, *e.g.*, by linear or non-linear amplification, attenuation, averaging, *etc.*;

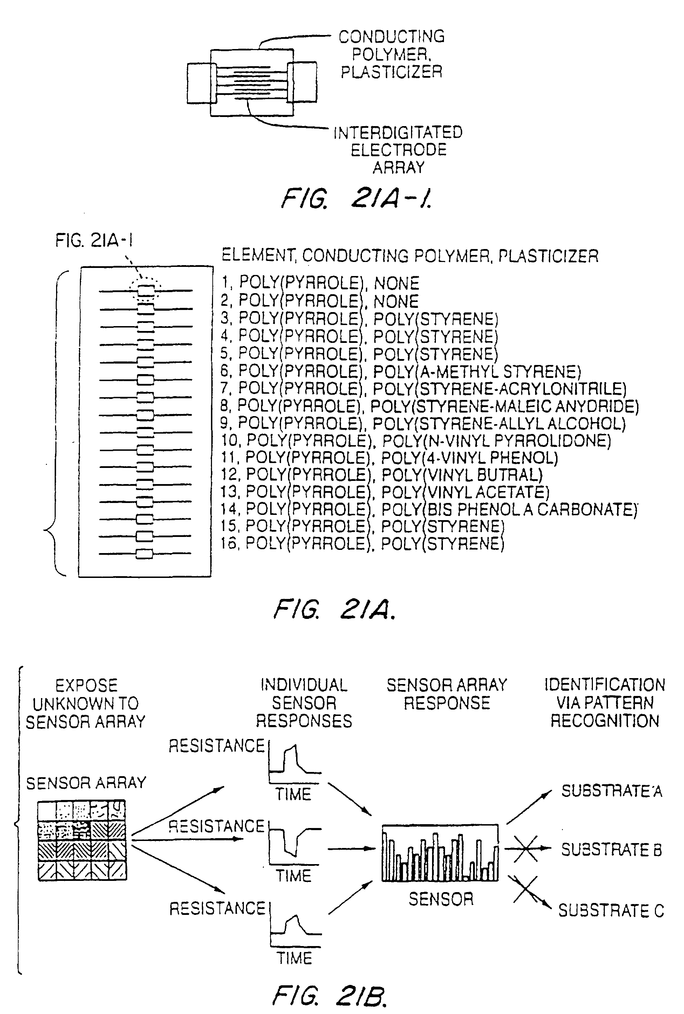

Figure 21(A) shows an overview of sensor design; Figure 21(B) shows an overview of sensor operation; Figure 21(C) shows an overview of system operation;

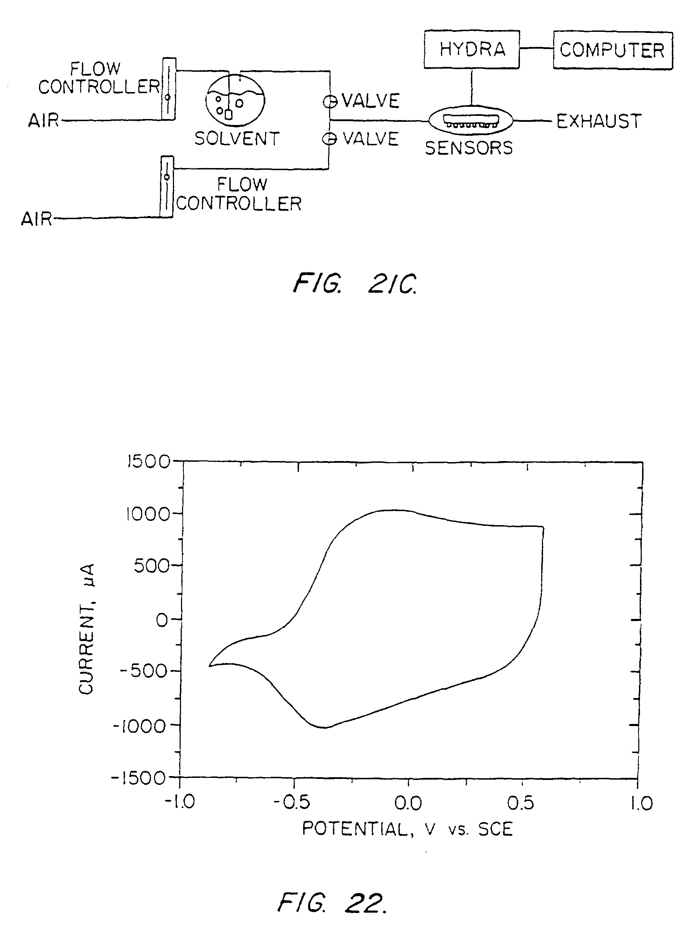

Fig 22. Cyclic voltammogram of a poly(pyrrole)-coated platinum electrode. The electrolyte was 0.10 M $[(C_4H_9)_4N]^+$ $[ClO_4]^-$ in acetonitrile, with a scan rate of 0.10 V s$^{-1}$;

Fig 23(A) shows the optical spectrum of a spin coated poly(pyrrole) film that had been washed with methanol to remove excess pyrrole and reduced phosphomolybdic acid. Fig. 23(B) shows the optical spectrum of a spin-coated poly(pyrrole) film on indium-tin-oxide after 10 potential cycles between +0.70 and -1.00 V vs. SCE in 0.10 M $[(C_4H_9)_4N]^-$ $[ClO_4]^-$ in acetonitrile at a scan rate of 0.10 V -s$^{-1}$. The spectra were obtained in 0.10 M KCl - $H_2O$;

Fig. 24(A) Schematic of a sensor array showing an enlargement of one of the modified ceramic capacitors used as sensing elements. The response patterns generated by the sensor array described in Table 3 are displayed for: Fig. 24(B) acetone; Fig 24(C) benzene; and Fig 24(D) ethanol;

Fig. 25. Principle component analysis of autoscaled data from individual sensors containing different plasticizers. (A) poly(styrene); (B) poly (?-methyl styrene); (C) poly(styrene-acrylonitrile); (D) poly(styrene-allyl alcohol);

Fig. 26(A) and 26(B). Principle component analysis of data obtained from all sensors (Table 3). Conditions and symbols are identical to Figs. 25(A)-25(D). Fig 26A shows data represented in the first three principle components pc1, pc2 and pc3, while Fig. 26B shows the data when represented in pc1, pc2, and pc4. A higher degree of discrimination between some solvents could be obtained by considering the fourth principle component as illustrated by larger separations between chloroform, tetrahydrofuran, and isopropyl alcohol in Fig. 26B.

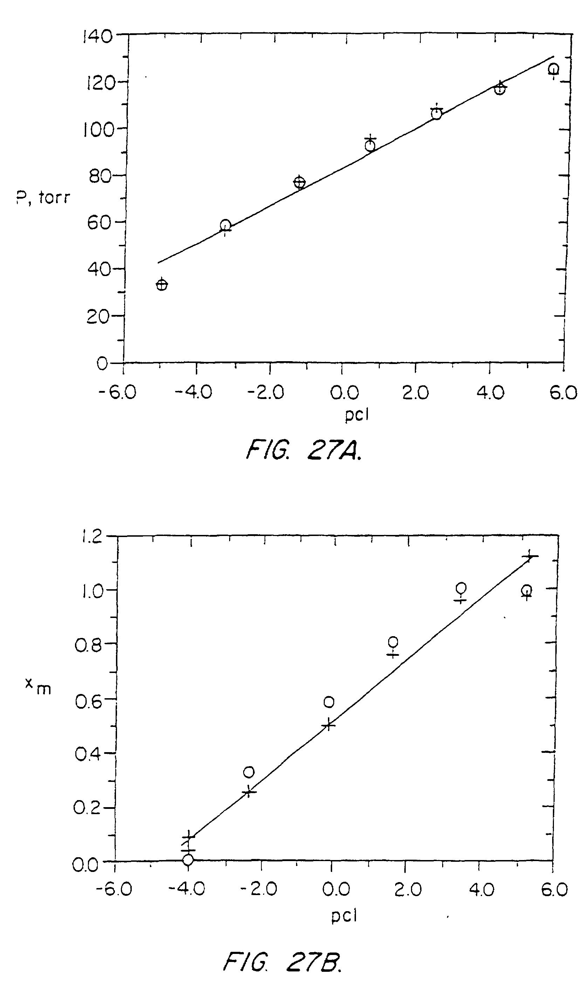

Fig. 27(A). Plot of acetone partial pressure (O) as a function of the first principle component; linear least square fit (-) between the partial pressure of acetone and the first principle component ($P_a$ = 8.26•pc1 + 83.4, $R^2$ = 0.989); acetone partial pressure (+) predicted from a multi-linear least square fit between the partial pressure of acetone and the first three principle components ($P_a$ = 8.26•pc1 - 0.673•pc2 + 6.25•pc3 + 83.4, $R^2$ = 0.998). Fig. 27(B). Plot of the mole fraction of methanol, $x_m$, (O) in a methanol - ethanol mixture as a function of the first principle component; linear least square fit (—) between $x_m$ and the first principle component ($x_m$ = 0.112•pc1 + 0.524, $R^2$ = 0.979); $x_m$ predicted from a multilinear least square fit (+) between $x_m$ and the first three principle components ($x_m$ = 0.112•pc1 - 0.0300•pc2 - 0.0444•pc3 + 0.524, $R^2$ = 0.987);

Fig. 28. The resistance response of a poly(N-vinylpyrrolidone):carbon black (20 w/w% carbon black) sensor element to methanol, acetone, and benzene. The analyte was introduced at t=60 s for 60 s. Each trace is normalized by the resistance of the sensor element (approx. 125?) before each exposure; and

Fig. 29. First three principal components for the response of a carbon-black based sensor array with 10 element. The non-conductive components of the carbon-black composites used are listed in Table 3, and the resistors were 20 w/w% carbon black.

## DESCRIPTION OF THE SPECIFIC EMBODIMENTS

The present invention provides techniques for the detection and identification of analytes. These analytes may be in fluids, which may be liquid or gaseous in nature. The techniques of the present invention may also be used to provide other information about analytes, including for example, the concentration, classification, volume, flow rate, direction of a plume trail, location of source of analyte, gradient, and other characteristics. For example, the techniques of the present invention may allow the determination of the concentration of a first analyte and a second analyte in a mixture.

A system of analyte detection of the present invention has many applications. This system may be embodied within analytical instruments, handheld devices, robots, and many other devices and tools. For example, the system of the present invention may, in a specific implementation, reside on a single integrated circuit or multiple integrated circuits. There are however many other ways to implement a system of the present invention- For example, the system of the present invention may have components which are relatively close in proximity to another, such as being resident on the same integrated circuit. Various components of the analyte detection system may also reside in different locations, and be linked by a network or other communications link. This network may include a local-area network, wide-area network, wireless network, cellular phone network, optical network, the internet, electrical wire, and many others, and combinations of these networks.

An example of a specific embodiment of the present invention is an electronic system of analyte detection. In particular, the electronic system of analyte detection may include a plurality of sensors. Further, one sensor in the' plurality of sensors may have a different characteristic from another sensor in the plurality. In an even further embodiment, each sensor in the plurality of sensors may have different characteristics from every other sensor. U.S. Patent Number 5,571,401 discusses sensors and sensor materials which may be used in a system of the present invention, although other sensors and sensor materials may also be used.

A technology that has led to the proliferation of modern electronics is the integrated circuit. Integrated circuit technology may be used in an electronic analyte detection system of the present invention. A system of the present invention may be contained within a handheld electronic device.

Using integrated circuit technology to fabricate an electronic analyte detection device permits relatively low cost and high volume manufacture of such devices. Integrated circuits are the modern marvel of today's electronic and information age. Commonly referred to as "chips", integrated circuits are miniaturized electronic circuits fabricated on silicon substrates. Chips are commonplace in the electronics market, and are the building blocks for a vast number of electronic products used in many industries. Products using integrated circuits include computers, computer peripherals, consumer electronics, telecommunications and networking equipment, and many others.

A system of the present invention may be manufactured using integrated circuit technology. The present invention is also not limited to electronic olfaction since a system according to the present invention may be used to detect, identify, and classify analytes in a variety of mediums and environments.

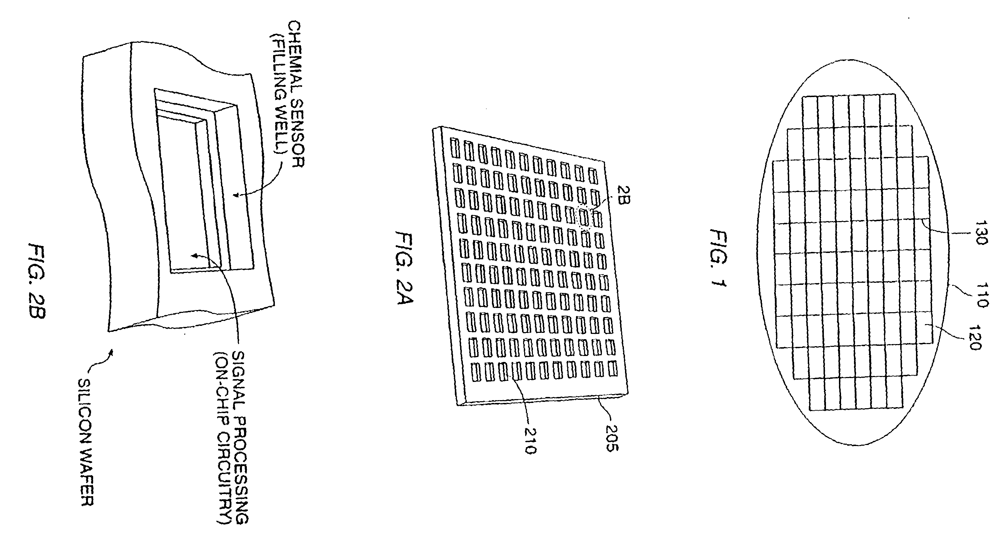

Figure 1 shows an implementation of the present invention using integrated circuit technology. A substrate or wafer 110 has a number of analyte detection chips 120. Similar to the case with integrated circuit fabrication, many analyte detection chips 120 may be formed on a single substrate. There may be hundreds or thousands of such chips on one substrate.

The substrate may be silicon, such as single crystal silicon having a < 1 0 0 > or < 1 1 1 > orientation. Other materials may also be used as a substrate including, just to name a few, other semiconductive materials, other materials suitable for the manufacture of integrated circuits, insulators, diamond, silicon (or other semiconductor material) over an insulator (such as sapphire), plastic, fused substrates, and polymers.

Analyte detection chips 120 may be fabricated on the substrate using a semiconductor process typical of the integrated circuit industry. Successive layers of various materials are formed and patterned on the substrate. The layers may include, just to name a few examples, diffusion (n-and p-type), silicon oxide, gate oxide, polysilicon, metal (including multiple layers of metal), contact, and via. These layers may be formed on the substrate by deposition, growth, ion implantation, sputtering, electroplating, and other techniques. Photoresist may be used to pattern the features on the substrate. Features may be etched using dry or wet etching techniques, and combinations of these in the same process.

In one embodiment of the present invention, analyte detection chips are fabricated using a CMOS process technology. Many other technologies may also be used, such as NMOS, BiCMOS, bipolar, and others.

Individual analyte detection chips are formed adjacent to other chips on the substrate. Individual chips are separated from each other by a scribe line 130. In many instances, each analyte detection chip is substantially identical to another. It is however possible to manufacture different types or different designs of analyte detection chips on a wafer. There may also be test die or structures on the wafer to allow testing and evaluation of various process parameters and properties of the analyte detection chips during the fabrication of the wafer. Test structures may also be formed in the scribe lines between the individual dies.

During the manufacture of analyte detection chips, a sensor material is placed on the substrate. For example, this sensor material may be deposited, coated, or otherwise applied on the substrate. In one embodiment, the sensor material is any material which provides an electrical response to an analyte or odorant. For example, an electrical response may be quantified in terms of impedance (R), inductance (L), capacitance (C), or other electrical changes. In an embodiment, the sensor material may be a polymer. The material may be organic, or inorganic in other embodiments. Further, the sensor material may consist of regions of a nonconductive organic material and a conductive material. In ocher embodiments, the sensor material may be insulating organic films that act as capacitors, or composite films that act as inductors. A more detailed description of some sensor materials and their properties is discussed in U.S. Patent Number 5,571,401. However, the present invention is not limited to the sensor materials in U.S. Patent Number 5,571,401 since other materials may also be used.

In a specific embodiment of the present invention, the sensor technology may involve a series of conductive polymeric composite vapor sensors. The presence of an analyte may be detected through a change in, for example, the electrical resistance of a chemically sensitive carbon-based resistor. As discussed above, changes in electrical properties other than resistance may also be used; these include the evaluation of capacitive and inductance changes.

Further, the sensor material may be composed of conductor and insulator composites. This material may be placed on the substrate in a film. The organic nonconducting polymer of the composite absorbs the analyte (which may be a vapor). This induces a change in the electrical properties of the sensor material. The sensor material may also undergo physical changes such as swelling. When the analyte is removed, any changes in the electrical properties reverse. For example, the resistance, capacitance, and inductance may return to their original value. Any physical changes would also reverse. The response of these types of sensors are reversible over multiple analyte exposures as well as reproducible over a large number of trials under a variety of ambient atmospheric conditions. Therefore, a device fabricated using these types of sensor materials would have a relatively long service life.

In the case of using a composite such a nonconducting polymer and carbon black, the sensor material will be temperature sensitive. When using temperature-sensitive sensors, the sensor should be kept at a relatively constant temperature to provide relatively consistent results. For example, a temperature such as about 5° C above the ambient should provide good results. Further, extremely high temperatures, say, above about 100° C, should be avoided since these temperatures would destroy the polymer sensor material or rapidly decrease its service life. For this reason, it is not expected that nonconducting polymer materials are to be used in the specialized environment of extreme high temperatures, say, from about 300° C to about 400° C or even higher. The polymer sensor materials will be usable in the normal temperature ranges from about 0° C to about 100° C.

Using a conductor and insulator composite for the sensor material permits a very broad, diverse collection of sensor materials. For example, any conducting element including carbon blacks, metallic colloids, or organic conducting polymers, and combinations of these, may be used as the conductive phase of the sensors. Any organic material may be used as the insulating phase of the sensors. Furthermore, an advantage of these types of sensor materials is that they do not have the stability limitations of conducting organic polymeric materials. A conductor and insulator composite also does not suffer the limitations from the types of substituents or restrictions on the ranges of swelling variations that can be obtained from backbone modification of pure organic conducting polymers.

After processing of a substrate or wafer is complete, the wafer is tested to determine the number and location of the "good" die. The percentage of good die on one wafer compared to the total number of die on the wafer is referred to as the "yield." Individual analyte detection dies are separated by sawing along the scribe lines. The analyte detection dies are then packaged, and may be further tested to ensure their proper operation. These dies may be packaged in a variety of packaging material including ceramic, epoxy, plastic, glass, and many others. Packaged analyte detection die may very much resemble packaged integrated circuit chips. For some types of applications, nonporous, nonreactive materials like ceramic may be used.

In one embodiment, the sensor material is deposited or applied at the wafer level, before individual dies are separated. In other embodiments, the sensor material is applied after the dies are separated.

Figure 2A shows a more detailed diagram of an analyte detection chip 205. In a basic embodiment, an analyte detection chip includes a plurality of sensor sites 210 of sensor material. The sensor material may be constrained by some means at each sensor site. There are many techniques of constraining the sensor material at specific sites on the substrate. For example, the sensor material may be constrained at specific sites by surface tension. The sensor material may also be constrained by an electrical charge, electric field, or magnetic field. Further, the sensor material may be constrained using structures formed by integrated circuit processing techniques or other techniques (e.g. micromachining or microelectromechanical systems (MEMS)).

In the specific example shown in Figures 2A and 2B, sensor wells, which are not according to the present invention, are used to constrain the sensor materials at the sensor sites. Figure 2B shows a more detailed view of a single sensor well. In the typical case, the sensor material may be deposited in the sensor wells of the analyte detection chips at the wafer level, before the chips are separated from the wafer. The sensor wells, however, may also be filled after the individual chips have been separated from the wafer.

For the analyte detection chip in Figure 2A, the sensor sites are arranged in an array having rows and columns of 11 sensor sites by 11 sensor sites, for a total of 121 sensor sites. As discussed above, the sensor sites in Figure 2A are sensor wells. Sensor material will be applied at these sensor sites which will serve as the analyte detection sensors.

The analyte detection chip depicted in the figure will have 121 sensors. In other embodiments, the analyte detection chip may have fewer than 121 sensors. For example, an analyte detection chip may have a two sensor sites, three sensor sites, four sensor sites, or greater number of sensor sites. An analyte detection chip may have two, three, four, five, six, seven, or more sensors sites for sensors. The chip may have ten to twenty, twenty to thirty, thirty to forty, forty to fifty, and fifty to one hundred sensors. A specific embodiment of the analyte detection chip has thirty-two sensor sites. Even more complex analyte detection chips may have many hundreds or thousands of sensors. For example, a chip may have 10,000 sensors (possibly arranged in an array with 100 sensors per side).

The array of sensors may be arranged in many possible formats, and may have an equal number of sensors per side. The arrangement of the plurality of sensor sites may be selected as appropriate for a particular application. Although Figure 2A shows a square array arrangement of sensor sites, the sensor sites may be arranged in any fashion on the chip. For example, the plurality of senior sites may be arranged in an oblong or rectangular structure, triangular structure, circular or curved structure, and many other arrangements. An array of sensor sites may have 1 site by 10 or more sites, 2 sites by 10 or more sites, 3 sites by 10 or more sites, 10 sites by 20 sites, or 30 by 175 sensors, just to mention some examples. There may also be multiple arrays or multiple groupings of sensor sites on the same substrate. There may be two, three, four, five, or more arrays of sensors on a single substrate.

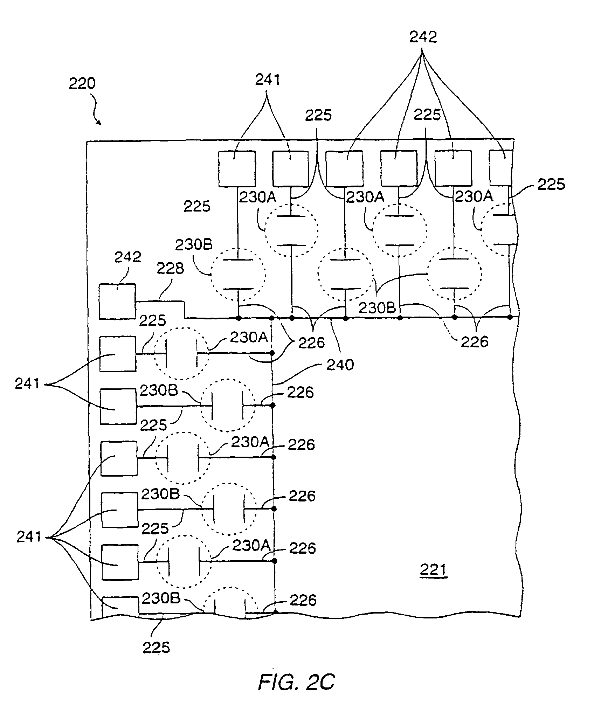

Figure 2C illustrates one embodiment of the present invention in which a detection chip 220 is formed with only a single conducting layer formed over a substrate 221- The single conducting layer, typically of metal, such as aluzixlum and its compounds, advantageously allows for a simple semiconductor process. The simpler processing provides for quicker manufacturing times and a reduced number of failure mechanisms. On the other hand, the simpler processing creates constraints in the layout of the chip 220 and necessarily creates a chip with some functional simplification.

The chip 220 provides for a number of sensors 230A and 230B around the periphery of the substrate 221. Only one corner of the substrate 221 is shown. The sensors 230A and 230B are arranged in two rows and are representationally illustrated by a dotted circle and two spaced-apart and parallel line segments. The dotted circle represents sensor material and the two line segments represent the electric terminals by which a reaction of an electrical parameter of the sensor material to an analyte or odorant is received. Each terminal is connected to one of two conductive leads 225 and 226, one lead 226 connected to a common line 240, i.e., a reference line, and the other lead 225 connected to a bonding pad 241. The common line 240 is arranged as a annular ring around the substrate 221 on the inside of the peripheral rows of the sensors 220A and 220B. By a lead connection 228 to a bonding pad 242, the voltage level of the common line 240 is fixed. As seen in Figure 2C, the two rows of sensors 230A and 230B are arranged in staggered fashion which allows the optimum packing of the sensors. The dotted circle of each sensor 230A and 230B also indicates the possible area covered by the sensor material described previously.

This arrangement permits electrical signals from each sensor 230 through the sensor's bonding pad 241 and the common bonding pad 242. The signals may be derived directly from the electrical characteristics of the sensor material or may be signals which have been preprocessed by the electrical circuits associated with each sensor 230, as described below. In either case, this arrangement can be implemented by "a single-metal layer" process, a term well understood in the semiconductor industry. Processing and layout is advantageously straightforward. With semiconductor technology readily available today, a chip with 32 sensors is easily manufactured. The surface is treated with gold to assure good contacts.

In other embodiments, a system of analyte detection may use sensors that reside on separate substrates. For example, the analyte detection system may gather analyte information from sensors in different physical locations such as sensors located at various positions of a production line or different rooms within a building.

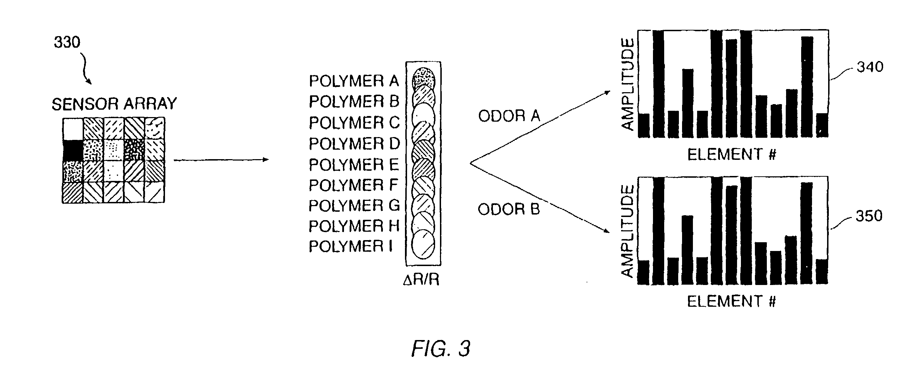

Figure 3 shows how a plurality of sensors 330 of the present invention may be used to identify an analyte. In an embodiment, the sensors would be formed on a substrate at sensor sites. and these sites may be arranged in an array form as discussed above. Each of the sensors may be incrementally different, and each is not specifically responsive to any particular analyte. For example, each sensor may have essentially a different polymer composite resistance change (listed as polymer A through polymer I) from every other sensor. When two analytes, such as odor A and odor B, are evaluated using the collection of sensors, the result will be two different response patterns 340 and 350. Each analyte has a characteristic "fingerprint." Pattern recognition processing may then be used to identify the analytes on the basis of these patterns.

In an embodiment of the present invention such as shown in Figure 3, every sensor has a different composition of sensor material from every other sensor. This may be referred to as "sensor diversity." In other embodiments of the present invention, however, there may be multiple sensors in a sensor array that are the same. In other words, some groups of sensors in this embodiment will be manufactured with exactly the same composition. while other groups of sensors will have a different composition. Having two or more of the same sensors in a sensor array may serve a redundancy purpose, which may be useful to increase the production yield. Redundancy in sensors may be useful for increasing the service life or reliability of an analyte detection chip, especially when used in harsh environments (e.g., industrial) or mission critical situations (e.g., military, bomb detection, or use by a common carrier). The techniques of the present invention for analyte detection also apply to cases where similar sensors exist in an array of sensors.

An aspect of the present invention is the use of a plurality of sensors having different response characteristics to distinguish and classify analytes. These sensors may be formed on the same substrate. The plurality of sensors will give a multidimensional response for use in characterizing and classifying the analyte.

A particular sensor material may be broadly responsive in the presence of many analytes. A response or signal from one sensor allows detection of a change in the composition of an analyte, but does not necessarily allow identification of that analyte. An array of sensor elements provides a reversible, diagnostic pattern of changes in an electrical parameter (such as resistance, capacitance, or inductance) upon exposure to different analytes. When a number of sensors with diverse chemical compositions is used, an analyte will have a particular fingerprint or signature.

Correlations between the elements of a sensor array may require many more than two sensors to successfully distinguish molecules in a complex environment. A greater number of sensors generally allows the identification of a greater number of analytes. Moreover, a greater number also decreases the chance that two analytes will have a similar or the same fingerprint. The sensitivity of an analyte detection system depends in part on the number of sensors, and diversity of the sensors. The analyte detection system of the present invention may be related to a biological analog, the nose. It is believed the human olfaction system has about $10^6$ total sensors of about $10^3$ different types of receptors. As is well known, dogs have a keener sense of smell than humans. A canine's nose has about $10^8$ sensors, which is two orders of magnitude greater then the human nose.

Greater numbers of sensors may be useful in a number of ways. It may be beneficial to measure the same property in many different ways due to noise limitations in a practical system. For example, if sufficient precision could be obtained, it might be possible to identify uniquely any molecule merely from a 38-bit measurement using two sensors. But in practice, it may not practical to make such precise measurements. Hence, when using lower precision measurements, useful information on the nature of the analyte may be obtained by making measurements using many independent determinations from many different sensor elements (such as in a sensor array).

Furthermore, a limited number of sensors may be sufficient to distinguish between a series of pure substances that are maintained at a fixed, known concentration. However, if the background is unknown, if mixtures are present, or if the background gases are changing in concentration, many more sensors may be needed simply to avoid ambiguity in interpretation of the output signal pattern. Even more sensors may be needed if optimal discrimination is to be accomplished between a given target signature and a wide possible range of background clutter. Having large numbers of sensors also allows redundancy and provides the ability to reject or veto the output of poorly performing sensors.

Having greater numbers of sensors may also improve a signal-to-noise response or reduce the time required to identify an analyte. It is possible to achieve signal-to-noise ratio gains from averaging over a large number of sensors during a given observation time. Therefore, with 10,000 sensors, for example, a $n^{1/2}$ signal-to-noise ratio gain would yield an effective sensitivity increase of almost two orders of magnitude over the capabilities of 1 to 10 sensors.

Because of all of these issues, the number of sensors to successfully sense and identify an analyte in a practical device may rapidly multiply from a minimum value. A main goal of array-based sensing is to insure that no two analytes will have the same fingerprint response from the array, and also that a given target pattern is not confused as a mixture of other, unanticipated or unknown, background components. Therefore, it is generally desirable to integrate large numbers of sensors into an array structure. The present invention permits the manufacture of a large number of sensor elements in a low-cost, parallel process. And, the processing allows sensor elements to be chemically diverse.

An array of six to eight sensors is sufficient to adequately distinguish between analytes. This is the case when the electronics used with the sensors provides adequate accuracy, such as a very precise analog-to-digital converter. As the number of sensors increases, fewer bits of accuracy will be required to distinguish between analytes as discussed above. For example, with sixteen to twenty sensors, less precise electronics are needed. With the integrated circuit technology available today, one practical implementation of an analyte detection chip has thirty-two sensors. Signals from thirty-two sensors may be decoded and processed by electronics using an analog-to-digital converter with about twenty bits of accuracy. This is not unduly complicated or prohibitively costly to implement. As integrated circuit technology improves, it is expected that it will become practical to fabricate more than thirty-two sensors on a single integrated circuit, and to process the signals from these sensors.

The chemical sensor material is applied at a sensor site. The chemical sensor material has electrical properties that can be measured in terms of electrical parameters. These parameters may be resistance, capacitance, or inductance. In the presence of an analyte or odor, the chemical sensor material will have a measurable response characteristic. A change or pattern of changes in the electrical properties of the sensors in sensor array may be measured to identify a particular analyte.

By evaluating a change in, for example, the resistance of the sensor material, an analyte detection system of the present invention may identify an analyte. A particular sensor may have a baseline resistance of 50K ohms (R1). However, when the sensor is placed in the presence of an analyte such as water vapor or hexane, the resistance of the sensor may change to 51K ohms (R2). This change in the resistance (i.e., (R1 - R2) - R1) relative to the baseline resistance value may be used to identify the analyte. The baseline resistance value is used as a reference point. The value of baseline resistance may vary depending on the operating conditions of the sensors such as the pressure, temperature, and humidity. The baseline resistance may also vary because the background ambient may change. For example, there may be background analytes which are not of interest and should not be considered during any measurements.

Changes in electrical properties other than resistance of the sensor material may also be measured and similarly analyzed. Resistance has been discussed merely as an example. A change in the capacitance or inductance of the sensor material may be measured to identify an analyte. In the presence of an analyte, the capacitance change of the sensor material (which may be due to a physical swelling of the material) may be measured.

A composition of the sensor material may determine its response characteristic. A sensor in a first position in the array may have a slightly different composition from another sensor in a second position in the array. The two sensors will give different response characteristics, and this difference may be used to help distinguish different analytes or odorants. For example, if a mixture of a nonconductive and conductive polymer is used as the sensor material for an array of sensors, the composition of the sensors may be different. In an embodiment where carbon black is used, the carbon black composition of each sensor may be slightly different from other sensors in the array.

In addition to the sensor sites for constraining the sensor material, the analyte detection chip of the present invention may include electrical or other connections to the sensor material at the sensor sites. For example, in the case when resistances of the sensors are to be evaluated, conductive layers such as metal may be used to connect with the sensor material in a similar fashion as metal interconnect is used in a semiconductor chip. In the case when capacitances are to be evaluated, a conductive material may be placed in proximity to the sensor material to allow capacitive coupling and sensing. The electrical signals from the sensor may then be routed to bonding pads of the analyte detection chip. Via the bonding pads, the electrical signals from the sensors may be connected to off-chip circuitry for further processing and analysis.

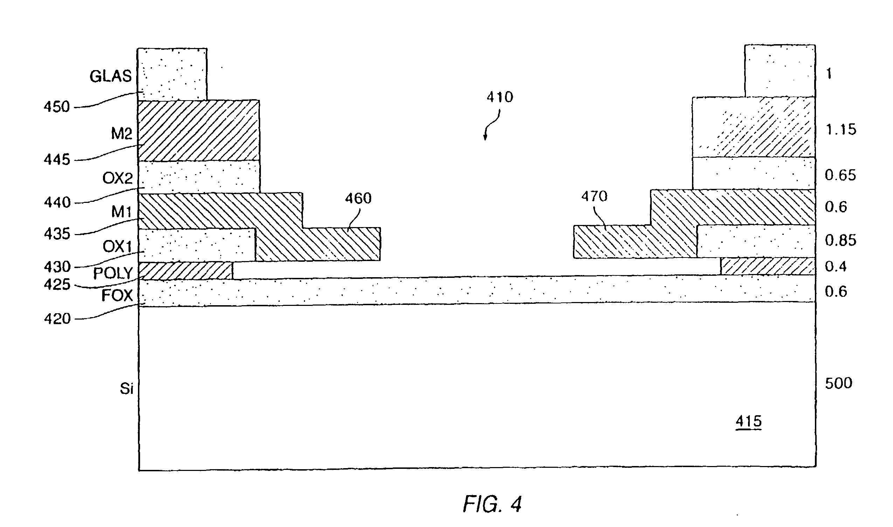

As discussed above, sensor wells constrain the sensor material. Figure 4 shows a cross section of an implementation of a sensor well not according to the present invention. This sensor well may be fabricated on a silicon substrate using a CMOS process. The sensor material will fill and be constrained by a sensor well 410. On a silicon substrate 415, the following layers may be patterned and used to form sensor well 410: a field oxide (fox) layer 420, a polysilicon (poly) layer 425, a first oxide (ox1) layer 430, a metal-1 (M1) layer 435, a second oxide (ox2) layer 440, a metal-2 (M2) layer 445, and a passivation or glass (GLAS) layer 450.

An example of a process flow for fabricating a sensor well is as follows. An oxide layer is formed over a silicon substrate. A metal or conductive layer is formed on the oxide layer. The metal layer is patterned and etched. The resulting metal is to be used as contacts for the sensor material. An oxide layer is formed on the structure. A sensor well is patterned and etched. The sensor material is deposited in the sensor well and is in electrical contact with the patterned metal layer.

The sensor material is applied to the sensor well after the sensor well is formed as a step during the fabrication of the chip (before the formation of the passivation layer). For example, the sensor material may be applied at the semiconductor fabrication facility. However, the sensor material may be applied in a postprocessing step, after the fabrication of the chip. For example, the sensor material is applied after the completed wafers are received from the semiconductor fabrication facility.

In one embodiment, the silicon substrate 415 is about 500 microns thick. The field oxide layer 420 is about 0.6 microns thick. The polysilicon layer 425 is about 0.4 microns thick. The first oxide layer 430 is about 0.85 microns thick. The metal-1 layer 435 is about 0.6 microns thick. The second oxide layer 440 is about 0.65 microns thick. The metal-2 layer 445 is about 1.15 microns thick. The passivation layer 450 is about 1 micron thick.

Although the structure in Figure 4 is fabricated using a two-layer metal process, a sensor well may be fabricated using a single-layer metal process and also processes having more than two layers of metal. For example, a sensor well may be fabricated in a process having three, four, five, or more layers of metal.

Electrical connections 460 and 470 are formed in the metal-1 layer to make electrical contact with the sensor material. These electrical connections are used to route the sensor signals to other circuitry for further processing of sensor data. This circuitry may be on-chip or off-chip. The metal conductor used to form connections 460 and 470 is typically a conductive material such as gold, platinum, aluminum, or copper. The material for the electrical connections 460 and 470 should be selected so they are not reactive to the sensor material. In the case when the sensor material is applied during a postprocessing step, connections 460 and 470 will be exposed, and a conductive material such as aluminum may easily oxidize. This may result in poor electrical connections to the sensor material.

Good electrical contacts are more important for some embodiments of the present invention than others. For example, a good physical contact may be important when measuring the resistance of the sensor material. This is especially true in cases when the sensor material has a relatively low resistance when compared to the contact resistance. In other cases, such as when measuring capacitance, connections may be made by using a capacitive connection, where there is no physical connection between the sensor material and the conductive material or metal. Consequently, in such an embodiment, there would be fewer concerns associated with oxidation of the metal connection.

The metal-1 layer may be, for example, postprocessed or at least finished in a nonstandard integrated circuit fashion. The surface of standard integrated circuit metalization is normally covered by a thin, air forming, "native" oxide layer. Aluminum, the most popular standard metal, forms aluminum oxide continuously over its surface very quickly when exposed to air. Polymer/carbon black composite resistors can not be taken to high temperatures nor can they be energetically formed in other ways to break through the "native" oxide, As such, a means for good contact to the metal layer must be made. This could be accomplished by chemically or physically etching the exposed electrodes and keeping the metal-1 in an oxygen-free environment while applying the polymer composite sensor material. More practically, an additional layer, or multiple layer sandwich, whose exposed layer is a noble (nonoxidizing) metal may be deposited through any number of techniques on the surface of metal-1. This technique could be physical vapor deposition or chemical vapor deposition or plating amongst others. The technique of sputtering a gold contact layer over a chromium glue layer, followed by photo lithographically defining the metal sandwich is especially attractive.

The circuitry receiving the sensor signals from connections 460 and 470 may be off-chip or on-chip. The other circuitry may include preprocessing, amplification, and classification of the sensor data. Depending on the packaging technology used, bonding pads may be formed along the periphery or edges of the chip, or may be distributed inside the chip (e.g., when using flip-chip packaging technology).

The sensor well structure of Figure 4 may be used to constrain and allow measurement of the sensor material. The sensor material fills or partially fills sensor well 410, and resistance is measured using electrical connections 460 and 470.

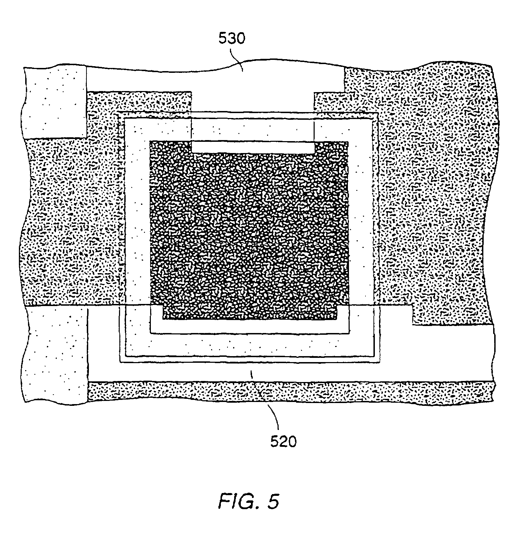

Figure 5 shows a top view of a 200-micron by 200-micron sensor well structure. Metal is used to make electrical connections 520 and 530 at opposite ends of the sensor well.

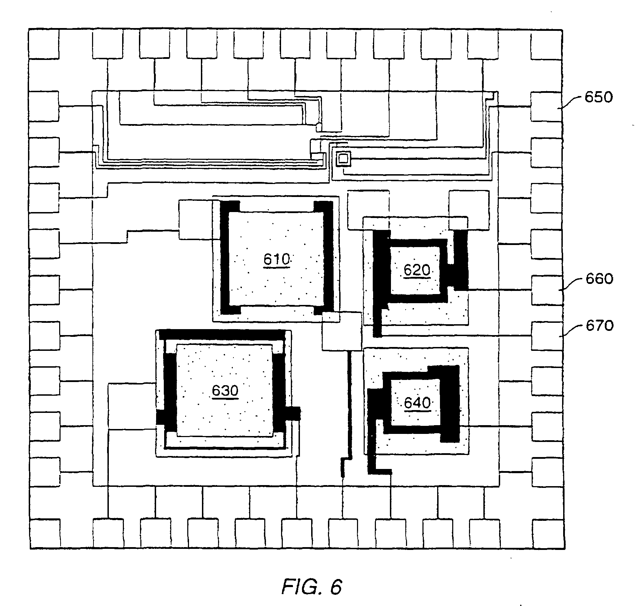

Figure 6 shows a layout of a test structure with four sensor wells 610, 620, 630, and 640. These sensor wells are of various sizes. Specifically, sensor wells 620 and 640 are squares of 200 microns per side while sensor wells 610 and 630 are squares of 400 microns per side. Bonding pads 650 surround the four sensor wells and are electrically connected to the sensor wells. Two bonding pads or electrical connections may be used to connect to a particular sensor well. For example, pads 660 and 670 connect to the two terminals for sensor well 620. One bonding pad or electrical connection may be shared between two different sensor wells.

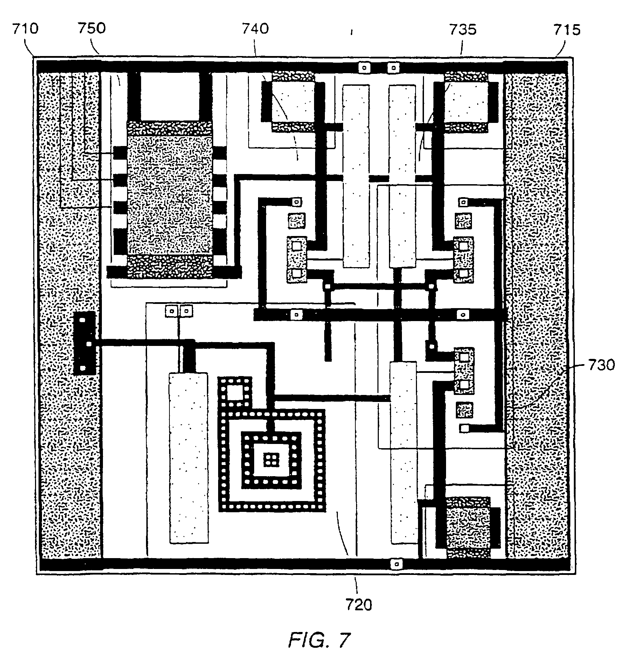

Figure 7 shows an embodiment not according to the present invention where electronic circuitry is formed below or beneath the sensor site. The figure shows a top view of a layout of the electronic devices at a sensor site. Electrical contacts 710 and 715 make electrical contact between the sensor material and electronic circuitry. In this case, the electronic devices implement a preprocessing circuit.

More specifically, the preprocessing circuit may include an autozeroing adaption circuit with signal amplification and X-Y decoding. The individual circuit blocks include a sensor read-out amplifier with baseline adaption circuit 720; signal amplification circuits 730, 735 and 740; and a row/column select and final output amplification circuit 750. In other embodiments, however, electronic circuitry for any purpose may be implemented at or beneath the sensor site. Outputs from the electronic circuitry may be routed to other on-chip circuitry, or off-chip circuitry via the bonding pads.

In Figure 7, the sensor site is a 200-micron by 200-micron sensor well. However, as discussed above, in an embodiment of the present invention, the sensor site comprises a sensor hurdle. Furthermore, in other embodiments, electronic circuitry is not necessarily formed beneath the sensor site, and may be placed anywhere on the same integrated circuit chip. For example, electronic circuitry may be formed adjacent to the sensors, or in another location on the chip. However, an advantage of forming electronic circuitry beneath the sensors is that the resulting layout is relatively compact.

A cross-sectional structure for the embodiment of Figure 7 may be similar to what is shown in Figure 4 where the electrical devices are formed using metal-1 and polysilicon layers. To be able to form electrical devices beneath the sensor well, the second oxide layer will not be etched through. The second oxide layer will instead form a "bottom" for the sensor well. The metal-1 layer is used to electrically connect to the sensor material at the sensor site.

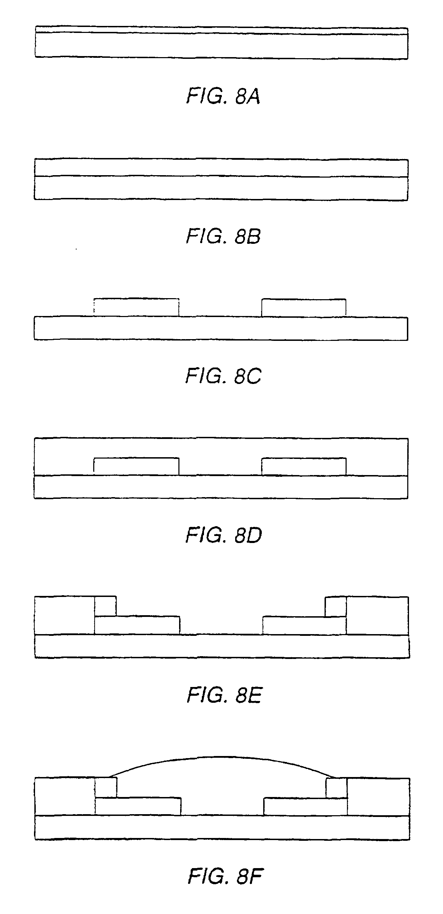

Figures 8A through 8F are not of the present invention and show the different stages in the microfabrication of a sensor well structure. The technique shown in Figure 8A through 8F may be an alternative to a CMOS semiconductor process. For example, the process may be a MEMS or microelectrical fabrication process or other specialised VLSI process. The process may include micromachining.

The process can be self-standing (with no underlying electronic circuits) or done in combination with other layers underneath the sequence of layers shown added in Figures 8A through 8F. A starting wafer or substrate is shown in 8A. This layer is either an insulating substrate or a starting wafer to which has been added an insulating film. This can be either through oxidation (for a silicon substrate) or deposition. A conductive film may be deposited onto the insulating surface by either physical or chemical vapor deposition methods shown in Figure 8B. The metal or conductive film is patterned in Figure 8C leaving a pair of electrodes. An additional insulating film is deposited in Figure 8D and patterned to expose the electrodes of a nonoxidized metal structure in Figure 8E. Into the well defined by the top insulator film and between the two electrodes in the bottom of the well, is deposited the sensor material shown in Figure 8F.

Sensor materials of diverse compositions are applied at the sensor sites of the chip. There are many techniques of applying the sensor material at the sensor sites. For example, the sensor material may be deposited at the sensor sites by using solution spin coating or deposition of monomers and then polymerizing them. In an embodiment where the sensor material are polymer-based chemiresistors, the polymer-based chemiresistors may be formed by spin- or dip-coating substrates with solutions or suspensions of the chemiresistor components. Furthermore, for the case of spin-coated layers or for the case of dip-coated layers, the need for diversity dictates there be a patterning of the first sensor material followed by the application and patterning of many subsequent layers. While not unfeasible, the number of times that this process need be repeated is dictated by the degree of diversity that is desired in the sensors.

Another technique to produce sensor sites containing sensor materials with diverse compositions is to deposit the sensor material serially in time. This will involve making a first deposition at a site which contains a distinct chemical composition from the second, from the third, and so forth.

A still further technique for applying the sensor material is to use microjet or ink jet technology. Ink jet technology is increasingly being used in the fabrication of devices. With such technology, it is possible to fabricate polymeric structures on the order of 100 microns and arrays of these structures with packing densities of greater than 15,000 per square centimeter. Microjets may be useful tools in fabricating large arrays of miniaturized sensors for analyte detection.

For example, to fabricate a diverse set of sensors on a substrate, a continuous jet system may be employed because the composition of the "ink" (e.g., the sensor material which may be a chemical polymer) can be continuously changed. This allows for the fabrication of sensor material films with variable composition from a limited feedstock of monomers or polymers as desired. The monomers delivered into the sensor sites would be polymerized in situ in a subsequent step through exposure to gamma irradiation, to a suitable free radical catalyst or by exposure to light. In this fashion, it will be possible to prepare libraries of thousands of different polymers from uncorrelated monomeric precursors, and to rapidly evaluate their efficacy in distinguishing the analytes of concern.

When using microjet technology, it is important to prevent the ink jet nozzles from clogging. It is desirable for the particle size of the ink be smaller than the nozzle size. In a specific embodiment, microjet technology may be used to apply polymers with carbon black. In fact, classic black inks (such as India ink) are carbon black suspensions. The nozzle size of commercial ink jets is generally greater than ten microns. Since a stable carbon black suspension with particle sizes of less than one micron may be formed, it is possible to fabricate carbon black suspensions compatible with microjet technology.

In addition to standard electrostatically controlled continuous flow or drop-on-demand systems, other options are available. Mechanically controlled ink jets with larger nozzles, essentially small spray guns, may also be used. Another microjet technology is the compound ink jet. With such a device, a jet of the so-called primary fluid emerges from a 10- to 20-micron orifice submerged in a so-called secondary fluid. The resulting jet consists of both fluids, and can be manipulated as in a standard electrostatically controlled continuous ink jet. Compound jets can utilize carbon black based inks, such as India ink, as a secondary fluid since the reservoir for this fluid can be of arbitrary size.

Although the above techniques for manufacturing are highly desirable for some applications, in other applications such as those that include a large numbers of sensor elements in the array, another embodiment may be more desirable. Fig. 7 shows a cross section of a portion of an integrated circuit 710 according to this further embodiment. An advantage of this method is that it is highly flexible, and might be used with any number of different base integrated circuit processes. For example, this method is especially useful for those applications which require addressing the array a bit at a time, because many such addressable array architectures have been developed in silicon technology, and this technique allows one to make use of these previously developed infrastructures.

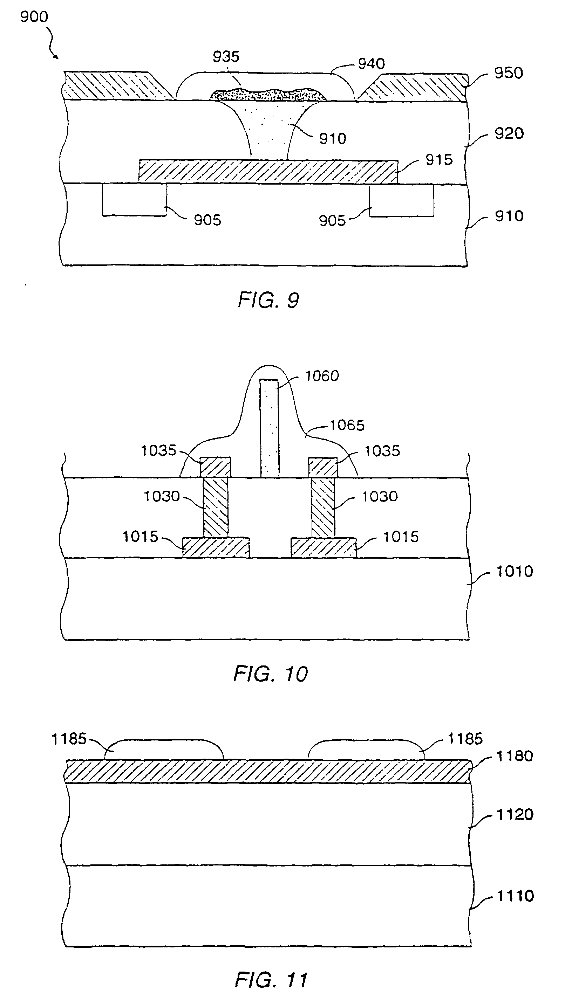

Referring to Figure 9, (not of the present invention) a plurality of semiconductor devices 905 are formed within a substrate 910 by any conventional VLSI fabrication processes, as is well-known in the art. conductors 915 are formed in a conductive layer to interconnect semiconductor devices 905 and to provide routing to the various sensors. Semiconductor devices 905 and conductors 915 are interconnected to form the various electronics on integrated circuit 900. For example, they may form the electronics for addressing and activating an array of sensor elements. Conductors 915 may be, for example, polysilicon, metal (e.g., aluminum or copper), or other conductive layers. In an embodiment of the invention that measures the change of resistance of a sensor, two layers of metal (not shown) are used for a bias to be generated and current measured at a given X-Y location in the array. Since the information that provides a signal is the change in the resistance of a node, the access lines can be relatively high impedance without causing any serious loss of signal or inducing much additional Johnson noise. Hence, the polysilicon layer, available in every CMOS technology, is usable. In a typical scenario, with a hundred squares of resistance at 10 ohms per square, a polysilicon line might be on the order 1000 ohms of fixed resistance in series with the signal resistor.

An insulator layer 920 of $SiO_3$, $SiOxN_4$, or other insulating material is formed above semiconductor device 905. Insulator layer 920 electrically isolates semiconductor devices 905 and conductors. 915. Contacts 910 are formed within insulator layer 920 to allow electrical connections to conductors 915, and may be formed of a variety of conductive materials such as tungsten or other refractory metals. Although only one contact 910 is shown in Figure 9 for simplicity, it will be recognized that each sensor may have more than one contact 910 connected to it, for example, to use the contact as a resistive element between two conductors.

After contacts 910 and insulator layer 920 are formed, integrated circuit 900 is planarized to provide a substantially flat surface. The planarization may be accomplished, for example, using chemical-mechanical processing (CMP), a technique well known in the art of integrated circuit processing. By so doing, contacts 910 are exposed. Contacts 910 having exposed metal may be covered with an optional noble metal coating 935 through physical vapor deposition, chemical vapor deposition, or plating techniques to provide an optimal electrical contact.

A polymer forming a sensor 940 of the type described above is deposited on contact 910 (or noble metal coating 935 if provided). In the combinatorial approach to making sensors devices, thousands of sensors 940 might be made by varying the composition of two, three, four, or more different types of polymers. A flat surface for this purpose would be desirable.

If for some reason a sensor well becomes necessary to physically separate individual sensors, beyond the electrical separation offered by the addressable contacts 930, then a second insulator layer 950 may be provided with opening for sensors 940. Insulator layer 950 is preferably Teflon® (a trademark of E.I. DuPont de Nemours and Company), Teflon®-like material, or other fluoropolymer, although other insulators may be used. In the case that postprocessing is needed, a flat topography on integrated circuit 900 from the planarization step is highly desirable.

Parcicularly in the case of an integrated circuit, a premium is placed on the amount of real estate taken up by sensors 940. To conserve real estate area, it is desirable to place the sensors above semiconductor devices 905 that make up the electronics for the array. This effectively doubles the use of the real estate. Because of their size, sensors 940 can become the determinant of the size of the chip if each sensor 940 has to be isolated physically from every other sensor 940: In this case, the dilution of the solution used to cast sensor 940 is desired to be as high as possible. The thinner the film the finer the degree to which it can be patterned or physically localized by other means.

In one embodiment of integrated circuit 900, sensor 940 should be as thin as possible without destroying its electrical properties. If the desired thickness of the polymer film becomes smaller than the conductive particle size, solution casting becomes impractical. Thus, in an alternate embodiment, sensors 940 are formed by putting the conductive particles down and then coating the conductive particles with the polymer films through a vapor deposition process. In some embodiments, these films may be made with no polymer at all, and yet still be sensitive to analytes. By putting down the conductive particles first and then coating them with a thin film of polymer, one could have an effective active film arbitrarily thin supported by the larger conductive particles in a porous configuration. Put another way, instead of casting a polymer film with included conductive particles, sensor 940 is formed by making a porous particle film with polymer coating the particles. This improves the response much faster and the lateral dimensions determined by the localization of a polymer vapor deposition.

Another response time enhancement is to make sensor 940 with an inert or sacrificial particle filler which is either very permeable or removable after deposition. While this does not change the thickness parameter positively, in some cases it is a simpler way to achieve the response time benefits of the spongy film detailed above with the application techniques that are in use today.

Figure 10 is a cross-sectional diagram shewing a sensor element according to the present invention created by another method. This technique also benefits from the planarized integrated circuit described with respect to Figure 9. In this embodiment, semiconductor devices, conductors 1015, and contacts 1030 are formed and the integrated circuit is planarized as described above, and optional noble metal coating 1035 is formed above contact 1030. Then, micromachining techniques are used to form high, hurdle-like structures 1060. By this technique, it is possible to place contacts 1030 very close together on the surface of the integrated circuit. A polymer film 1065 is deposited onto hurdle-like structure 1060 with a thickness that may be determined by the surface tension or wetting properties of the polymer, solvent conductive particle mixture, rathar than the volume in the drop or dispensed amount. This allows the sensor to be thinner, the response faster and the silicon area to be reduced.

Figure 11 (not of the present invention) shows another embodiment that may take advantage of the planarization technique described above with respect to figure 9. Above the planarized insulator layer 1120, a very high impedance film 1180 is placed across semiconductor devices (not shown in Figure 11) that form the array. Chemiseusitive sensors 1185 are deposited right on top of high impedance film 1180 forming a distributed parallel resistor. This allows working in a domain of thinness where the actual signal generating film does not need to be continuous. Even if short segments change, the terminal resistances within the array would be impacted.

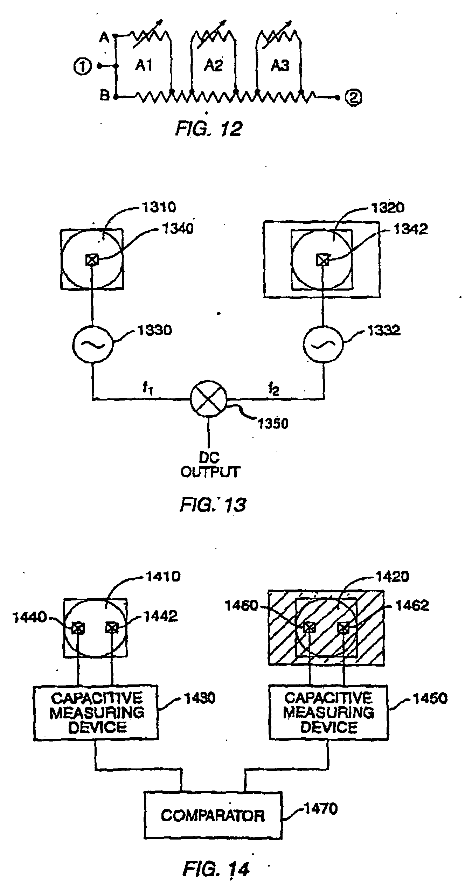

In particular, the equivalent circuit for the case of a discontinuous film on top of a continuous high-impedance film is shown in Figure 12. The high impedance film is represented by leg B in the drawing or as a continuous resistor. Leg A of the drawing shows a group of variable resistors that are in parallel with the underlying resistor. When the resistors A1 through A3 change in response to the presence of an analyte, the resistance between points 1 and 2 of the drawing change even though the changing film may not be continuous.

Figure 13 is a block diagram of an embodiment of the present invention that measures a capacitance of the sensor material to determine the presence of an analyte. While Figure 13 shows only a single pair of seniors, the circuitry may also be expanded to include an array of sensors or an array of pairs of sensors. Each sensor in the array may include a different type of sensor material from other sensors as described above.

Capacitance may be measured in a variety of ways. Figure 13 depicts one such method. However, other circuitry for measuring capacitance may be substituted for the circuitry shown. In the embodiment shown, two sensors 1310 and 1320 are provided. Sensors 1310 and 1320 are sensors formed substantially identical to one another. However, sensor 1310 is exposed such that analytes may penetrate the sensor material and cause it to expand. On the other hand, sensor 1320 is covered by an insulator layer so that it will not be affected by analytes. As such, sensor 1320 is a reference sensor, and its capacitance can be compared with the capacitance of sensor 1310 to determine if sensor 1320 has expanded due to the presence of an analyte.

One technique of evaluating the capacitors of the sensors involves frequency generators. Frequency generators 1330 and 1332 are coupled to sensors 1310 and 1320, respectively, through contacts 1340 and 1342. Frequency generators 1330 and 1332 output an oscillating signal at a particular frequency, and receive back return signals f1 and f2. Return signals f1 and f2 may be phase-shifted or frequency shifted, depending upon the capacitance of the sensor. Thus, if sensor 1310 has not expanded, the capacitance is the same as that of sensor 1320 and f1 is the same as f2. In the case when an analyte is present, the capacitance of sensor 1320 is greater, and thus f1 is not the same as f2. In fact, the difference between f1 and f2 may be used to determine the change in capacitance.

The return signals f1 and f2 are input to a discriminator mixer 1350. Discriminator mixers are well known in the electrical arts, and in particular for example, in the design of phase locked loops. Mixer 1350 receives two frequencies, and outputs a DC output that is zero if the frequencies are the same, and nonzero if the frequencies are different. The greater the frequency difference, the higher the value of the DC output. Thus, if the output of mixer 1350 is zero, then the capacitance of the two sensors are the same, and no analyte is present; if the magnitude of the output is nonzero, then an analyte is present, and may be identified by the value of the DC output.

Of course, other capacitance measuring circuitry may also be used. For example, two similar adjacent sensors may be formed such that they have room co expand in a sideways direction. Each of the two sensors are coupled to a different conductive trace, and the sensors are coupled through the conductive trace to a capacitance measuring circuit. When no analyte is present, the sensors have a certain separation, that is known, and thus has a known capacitance. When an analyte is present, the sensors expand and the distance between them shortens causing the capacitance to change. By measuring the change in capacitance, the presence of the analyte may be determined.

Figure 14 shows another embodiment of the present invention for measuring the capacitance of a sensor element. Two similar sensors 1410 and 1420 are provided. In a specific embodiment, sensors 1410 and 1420 are substantially identical. A capacitive measuring device 1430 coupled to sensor 1410 by two conductors 1440 and 1442 through contacts or otherwise. The capacitive measuring device is any device capable of determining a capacitance of sensor 1410. Similarly, a second capacitance measuring device 1450 is coupled to sensor 1420 through two conductors 1460 and 1462. Sensor 1420 is isolated from exposure to analytes, while sensor 1410 may be exposed to them. A comparator 1470 compares the capacitances measured from the two sensors 1410 and 1420. These values may be analyzed by various techniques described above or otherwise.

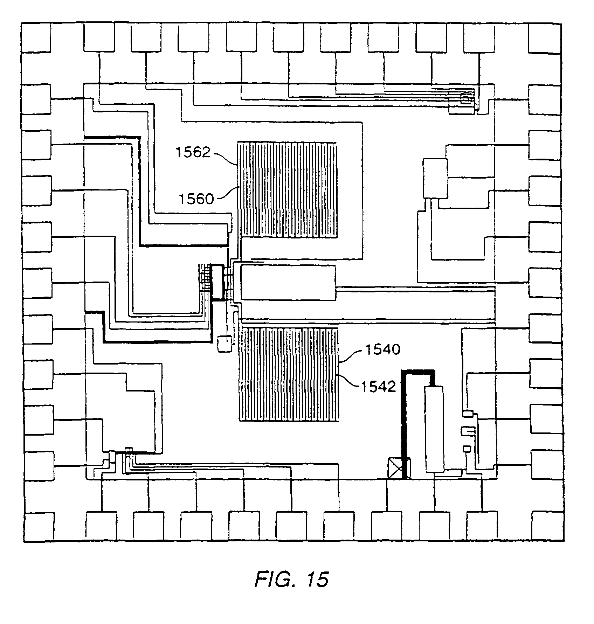

Figure 15 shows an integrated circuit layout that may be used for the circuit shown in Figure 14. Conductors 1540 and 1542 are interdigitated on the integrated circuit. These conductors are associated with one capacitor. A sensor (not shown)is formed above the interdigitated conductors. Similarly, another sensor (not shown) is formed above interdigitated conductors 1560 and 1562. These conductors are associated with another capacitor.

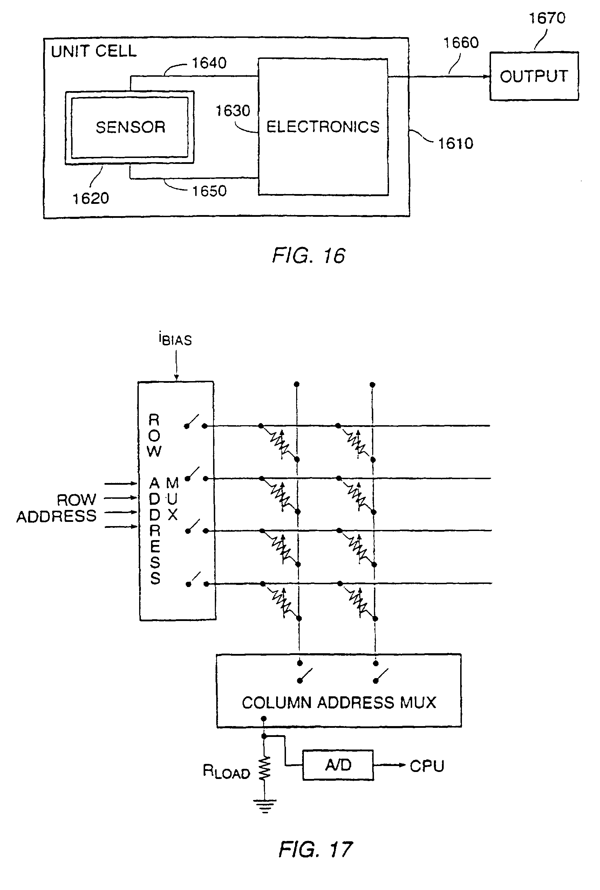

Figure 16 shows a "unit cell" 1610 for a sensor of the analyte detection chip of the present invention. To form a plurality of sensors, unit cell 1610 may be repeated as many times as desired. For example, for an analyte detection chip with ten sensors, the unit cell is repeated ten times. For an analyte detection chip with thirty sensors, the unit cell is repeated thirty times. For a chip with 100 sensors, the unit cell is repeated 100 times. For a chip with "n" sensors, the unit cell is repeated at least "n" times.

As discussed above, a basic embodiment of unit cell 1610 includes sensor 1620 by itself. Electrical connections from the unit cell will be connected to other electronic circuitry, on-chip or off-chip, for further processing. For example, in a two-chip analyte detection chipset solution, a first of the chips may contain a plurality of sensors 1620 and their respective electrical connections. A second of the chips may be electrically coupled to sensors 1620 to process the signals from the sensors on the first chip.

A more highly integrated embodiment of unit cell 1610 includes sensor 1620 and electronics 1630, both on the same chip or substrate. Electronics 1630 may be formed beneath the sensor site of sensor 1620, as was described for Figure 4 above. Electronics 1630 are electrically coupled to sensor 1620 by connections 1640 and 1650. Electronics 1630 processes the signals from the sensor. The processing includes amplification or filtering, or both. An output 1660 of the electronics may be coupled to other circuitry 1670 for even further processing. For example, the other circuitry may be off-chip for classifying the analyte.

Figure 17 shows an embodiment of circuitry for reading out a sensor array. As the number of wires grows with the number of sensors in an array, the practicality of using an inactive array is reduced. It becomes desirable as the number of sensors in the array approaches about 100 to reduce the wiring complexity with the addition of a matrix addressing scheme shown in Figure 17. The array of chemically sensitive sensors is shown in this embodiment as variable resistors, each connected between a row bias line and a column read line. A row and a column multiplexer are to "sample" the sensor data in a scheme somewhat like to scanning a television picture. A row address is translated into the application of bias (i.e., iBias) to one row, and the column address is translated into the closure of a column read switch switching the output to a load resistor that is at the input of an analog-to-digital (A/D) converter whose output is in turn fed to the controller of the system. It should be clear that the functions of bias and read could be reversed and that other configurations of lead resistors, included buffering circuitry and many other functions could be included.

In an embodiment of an array of sensor cells, there may also be dummy rows and columns of sensors, which is a row or column of sensors is formed but not used functionally as are active rows and columns of sensors. For example, at row and column edges of the array, dummy rows and columns of sensors may be formed. These dummy rows and columns of sensors may be used to ensure the active interior row and columns of sensors are relatively uniform, since sensors at the edge may exhibit some edge effects by not having a similar number of adjacent sensors as for the interior sensors.

Dummy rows and columns (not necessarily at edges of the array) may also be used in a redundancy scheme when these are activated, possibly by laser programming or programming of nonvolatile or one-time programmable memory elements such as Flash, EEPROM, EPROM, or antifuse cells. These dummy row and columns may be used in the place of other rows and columns that are or have become defective. For example, a redundancy scheme may help improve the yield of good die, or increase the service life of an analyte detection chip.

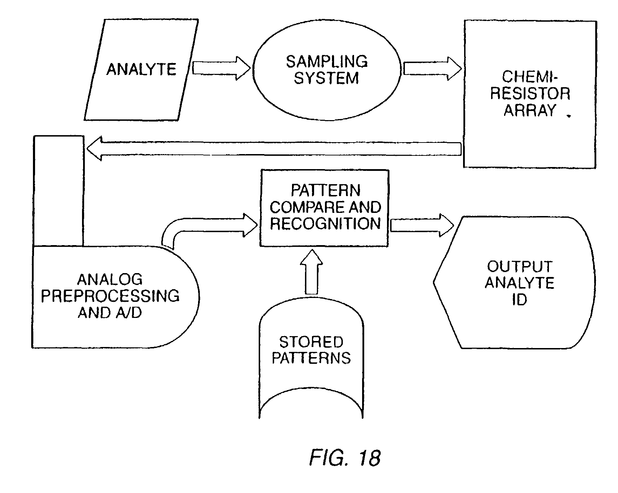

Figure 18 shows a block diagram of an analyte detection system. The block diagram for a discrete system that has been developed are shown in the analyte detection system block diagram and system design. Any full analyte sampling system should include a means for sampling the analyte of interest. This could be as simple as a stick to attach the sensors and a means for holding it in the vicinity of a vapor of interest, or as complex as a network of pumps and valves sequencing through a complex sampling routine. Once the analyte has been presented to the array of chemically sensitive transducers, the signals are processed and presented to an A/D converter. The pattern of response across the array is then compared to a stored pattern of response and an identification can be made through any number of possible input output channels as simple as wires to a control system or as complex as a visual display system.

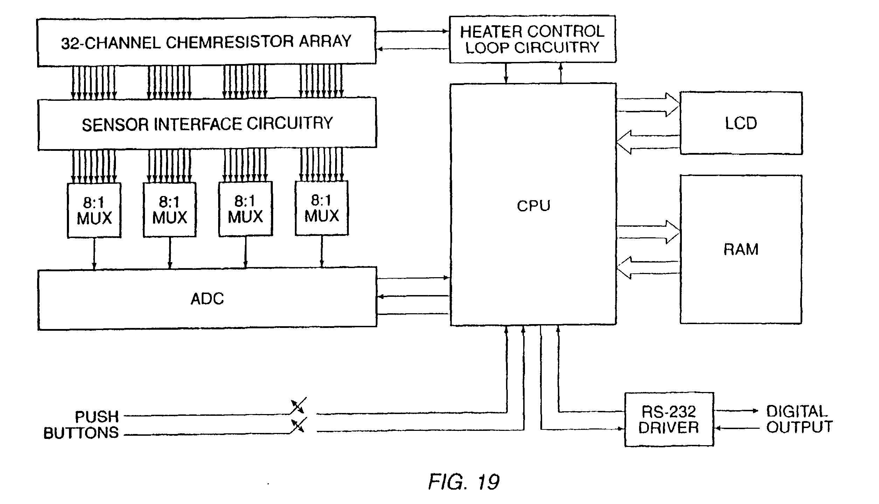

Figure 19 shows a block diagram of a specific embodiment of an analyte system of the present invention. A particular embodiment of such a system is shown in the block diagram of a system that has been implemented in a discrete design. There are thirty-two sensors (e.g., chemiresistive sensors) organized in four groups of eight. The signals are buffered, and each bank of eight sensor signals is then fed through an 8-to-1 analog multiplexer to an A/D that has an additional 4-to-1 multiplexer internal to it. The data is streamed out of the A/D converter in a serial bit stream to a central processing unit (CPU). The CPU may be a computer. The CPU additionally is interfaced to a heater control system. As the chemically sensitive sensors are also temperature dependant, controlling the temperature in the system eliminates one source of noise. The data is stored by the CPU in random access memory (RAM) or in another storage media such as magnetic disk. The measurements can be compared to a learned pattern of response previously stored and the CPU can calculate the best match and report the result through the LCD panel display.

As the number of sensors grows beyond thirty-two, the number of connections can become impractical to make with solder or other physical attachment processes. More of the block diagram will then be integrated onto a chip since the wiring connections inside and integrated circuit are very reliable. As the number of sensors approaches 100, it makes economic sense to integrate a matrix measurement scheme on the same substrate as the sensors. As the number of sensors grows even further, the A/D converter can become overtaxed and more than one makes sense to keep the system throughput in the range of one second where the flow system time constant becomes the limitation to overall system response. As the number of sensors grows to an even larger number, the A/D technology needs to be changed to either a large array of slower A/Ds or a faster variety of converter or both. With an array of A/D converters on the chip a digital multiplexer needs to be added to funnel the outputs through to the CPU. As the number of sensor elements climbs to the millions, some condensation of the data needs to take place within the array itself.
The following examples are set out below to supplement the specificity of the above examples.

The device includes a chemical sensor comprising first and second conductive elements (*e.g.*, electrical leads) electrically coupled to a sensor material in the form of a chemically sensitive resistor which provides an electrical path between the conductive elements. The resistor comprises a plurality of alternating nonconductive regions (comprising a nonconductive organic polymer) and conductive regions (comprising a conductive material). The electrical path between the first and second conductive elements is transverse to (*i.e.*, passes through) said plurality of alternating nonconductive and conductive regions. In use, the resistor provides a difference in resistance between the conductive elements when contacted with a fluid comprising a chemical analyte at a first concentration, than when contacted with a fluid comprising the chemical analyte at a second different concentration.

The electrical path through any given nonconductive region is typically on the order of 100 angstroms in length, providing a resistance of on the order of 100 m$\Omega$ across the region. Variability in chemical sensitivity from sensor to sensor is conveniently provided by qualitatively or quantitatively varying the composition of the conductive and/or nonconductive regions. For example, in one embodiment, the conductive material in each resistor is held constant (*e.g.*, the same conductive material such as polypyrrole) while the nonconductive organic polymer varies between resistors (*e.g.*, different plastics such as polystyrene).

Arrays of such sensors can be constructed with at least two sensors having different chemically sensitive resistors providing dissimilar differences in resistance. An electronic nose for detecting an analyte in a fluid can be constructed by using such arrays in conjunction with an electrical measuring device electrically connected to the conductive elements of each sensor. Such electronic noses may incorporate a variety of additional components including means for monitoring the temporal response of each sensor, assembling and analyzing sensor data to determine analyte identity, etc. Methods of making and using the disclosed sensors, arrays and electronic noses are also described herein.

Sensor arrays for detecting an analyte in a fluid for use in conjunction with an electrical measuring apparatus comprise a plurality of compositionally different chemical sensors. Each sensor comprises at least first and second conductive leads electrically coupled to and separated by a chemically sensitive resistor. The leads may be any convenient conductive material, usually a metal, and may be interdigitized to maximize signal-to-noise strength.

The remainder of this discussion addresses an embodiment where the electrical property of interest for the chemical sensor material is resistance. However, it is also recognized that the chemical sensor material has other electrical properties including capacitance and inductance. The sensor material exhibits a swelling phenomenon that may be measured by changes in any electrical property such as resistance, capacitance, or inductance, or combinations of these properties. For example, as discussed above, in other embodiments of the present invention, detecting analytes may be accomplished by evaluating a capacitance of the sensor material.

The resistor comprises a plurality of alternating nonconductive and conductive regions transverse to the electrical path between the conductive leads. Generally, the resistors are fabricated by blending a conductive material with a nonconductive organic polymer such that the electrically conductive path between the leads coupled to the resistor is interrupted by gaps of non-conductive organic polymer material. For example, in a colloid, suspension or dispersion of particulate conductive material in a matrix of nonconductive organic polymer material, the matrix regions separating the particles provide the gaps. The nonconductive gaps range in path length from about 10 to 1,000 angstroms, usually on the order of 100 angstroms providing individual resistance of about 10 to 1,000 m$\Omega$, usually on the order of 100 m$\Omega$, across each gap. The path length and resistance of a given gap is not constant but rather is believed to change as the nonconductive organic polymer of the region absorbs, adsorbs or imbibes an analyte. Accordingly the dynamic aggregate resistance provided by these gaps in a given resistor is a function of analyte permeation of the nonconductive regions. In some embodiments, the conductive material may also contribute to the dynamic aggregate resistance as a function of analyte permeation (*e.g.*, when the conductive material is a conductive organic polymer such as polyprryole).

A wide variety of conductive materials and nonconductive organic polymer materials can be used. Table 1 provides exemplary conductive materials for use in resistor fabrication; mixtures, such as of those listed, may also be used. Table 2 provides exemplary nonconductive organic polymer materials; blends and copolymers, such as of the polymers listed here, may also be used. Combinations, concentrations, blend stoichiometries, percolation thresholds, *etc.* are readily determined empirically by fabricating and screening prototype resistors (chemiresistors) as described below.

Table 1.

| Major Class | Examples |
|---|---|
| Organic Conductors | conducting polymers (poly(anilines), poly(thiophenes), poly(pyrroles), poly (acetylenes), *etc.*)), carbonaceous materials (carbon blacks, graphite, coke, $C_{60}$, *etc.*), charge transfer complexes (tetramethylparaphenylenediami ne-chloranile, alkali metal tetracyanoquinodimethane complexes, tetrathiofulvalene halide complexes, *etc.*), etc. |
| Inorganic Conductors | metals and metal alloys (Ag, Au, Cu, Pt, AuCu alloy, *etc.*), highly doped semiconductors (Si, GaAs, InP, $MoS_2$, $TiO_2$, *etc.*), conductive metal oxides ($In_2O_3$, $SnO_2$, $Na_xPt_3O_4$, *etc.*), superconductors ($YBa_2Cu_3O_7$, $Tl_2Ba_2Ca_2Cu_3O_{10}$, etc.), *etc.* |
| Mixed inorganic/organic Conductors | Tetracyanoplatinate complexes, Iridium halocarbonyl complexes, stacked macrocyclic complexes, *etc.* |

Table 2.

| Major Class | Examples |
|---|---|
| Main-chain carbon polymers | poly(dienes), poly(alkenes), poly(acrylics), poly(methacrylics), poly (vinyl ethers), poly(vinyl thioethers), poly(vinyl alcohols), poly(vinyl ketones), poly(vinyl halides), poly(vinyl nitriles), poly(vinyl esters), poly (styrenes), poly(arylenes), *etc.* |
| Main-chain acyclic heteroatom polymers | poly(oxides), poly(carbonates), poly(esters), poly(anhydrides), poly (urethanes), poly(sulfonates) , poly(siloxanes), poly(sulfides), poly (thioesters), poly(sulfones), poly(sulfonamides), poly(amides), poly (ureas), poly(phosphazenes), poly(silanes), poly(silazanes), *etc.* |
| Main-chain Heterocyclic polymers | poly(furan tetracarboxylic acid diimides), poly(benzoxazoles), poly (oxadiazoles), poly(benzothiazinophenothiaz ines), poly (benzothiazoles), poly(pyrazinoquinoxalines), poly(pyromellitimides), poly(quinoxalines), poly(benzimidazoles), poly(oxindoles), poly (oxoisoindolines), poly(dioxoisoindolines), poly(triazines), poly (pyridazines), poly(piperazines), poly(pyridines), poly(piperidines), poly (triazoles), poly(pyrazoles), poly(pyrrolidines), poly(carboranes), poly (oxabicyclononanes), poly(dibenzofurans), poly(phthalides), poly (acetals), poly(anhydrides), carbohydrates, *etc.* |

The chemiresistors can be fabricated by many techniques such as, but not limited to, solution casting, suspension casting, and mechanical mixing. In general, solution cast routes are advantageous because they provide homogeneous structures and ease of processing. With solution cast routes, resistor elements may be easily fabricated by spin, spray or dip coating. Since all elements of the resistor must be soluble, however, solution cast routes are somewhat limited in their applicability. Suspension casting still provides the possibility of spin, spray or dip coating but more heterogeneous structures than with solution casting are expected. With mechanical mixing, there are no solubility restrictions since it involves only the physical mixing of the resistor components, but device fabrication is more difficult since spin, spray and dip coating are no longer possible. A more detailed discussion of each of these follows.

For systems where both the conducting and non-conducting media or their reaction precursors are soluble in a common solvent, the chemiresistors can be fabricated by solution casting. The oxidation of pyrrole by phosphomolybdic acid presented herein represents such a system. In this reaction, the phosphomolybdic acid and pyrrole are dissolved in tetrahydrofuran (THF) and polymerization occurs upon solvent evaporation. This allows for THF soluble non-conductive polymers to be dissolved into this reaction mixture thereby allowing the blend to be formed in a single step upon solvent evaporation. The choice of non-conductive polymers in this route is, of course, limited to those that are soluble in the reaction media. For the poly(pyrrole) case described above, preliminary reactions were performed in THF, but this reaction should be generalizable to other non-aqueous solvent such as acetonitrile or ether. A variety of permutations on this scheme are possible for other conducting polymers. Some of these are listed below. Certain conducting polymers, such as substituted poly-(cyclooctatetraenes), are soluble in their undoped, non-conducting state in solvents such as THF or acetonitrile. Consequently, the blends between the undoped polymer and plasticizing polymer can be formed from solution casting. After which, the doping procedure (exposure to $I_2$ vapor, for instance) can be performed on the blend to render the substituted poly(cyclooctatetraene) conductive. Again, the choice of non-conductive polymers is limited to those that are soluble in the solvents that the undoped conducting polymer is soluble in and to those stable to the doping reaction. Certain conducting polymers can also be synthesized via a soluble precursor polymer. In these cases, blends between the precursor polymer and the non-conducting polymer can first be formed followed by chemical reaction to convert the precursor polymer into the desired conducting polymer. For instance poly(*p*-phenylene vinylene) can be synthesized through a soluble sulfonium precursor. Blends between this sulfonium precursor and the non-conductive polymer can be formed by solution casting. After which, the blend can be subjected to thermal treatment under vacuum to convert the sulfonium precursor to the desired poly(*p*-phenylene vinylene).

In suspension casting, one or more of the components of the resistor is suspended and the others dissolved in a common solvent. Suspension casting is a rather general technique applicable to a wide range of species, such as carbon blacks or colloidal metals, which can be suspended in solvents by vigorous mixing or sonication. In one application of suspension casting, the non-conductive polymer is dissolved in an appropriate solvent (such as THF, acetonitrile, water, *etc.*). Colloidal silver is then suspended in this solution and the resulting mixture is used to dip coat electrodes.

Mechanical mixing is suitable for all of the conductive/non-conductive combinations possible. In this technique, the materials are physically mixed in a ball-mill or other mixing device. For instance, carbon black : non-conductive polymer composites are readily made by ball-milling. When the non-conductive polymer can be melted or significantly softened without decomposition, mechanical mixing at elevated temperature can improve the mixing process. Alternatively, composite fabrication can sometimes be improved by several sequential heat and mix steps.

Once fabricated, the individual elements can be optimized for a particular application by varying their chemical make up and morphologies. The chemical nature of the resistors determines to which analytes they will respond and their ability to distinguish different analytes. The relative ratio of conductive to insulating components determines the magnitude of the response since the resistance of the elements becomes more sensitive to sorbed molecules as the percolation threshold is approached. The film morphology is also important in determining response characteristics. For instance, thin films respond more quickly to analytes than do thick ones. Hence, with an empirical catalogue of information on chemically diverse sensors made with varying ratios of insulating to conducting components and by differing fabrication routes, sensors can be chosen that are appropriate for the analytes expected in a particular application, their concentrations, and the desired response times. Further optimization can then be performed in an iterative fashion as feedback on the performance of an array under particular conditions becomes available.

The resistor may itself form a substrate for attaching the lead or the resistor. For example, the structural rigidity of the resistors may be enhanced through a variety of techniques: chemical or radiation cross-linking of polymer components (dicumyl peroxide radical cross-linking, UV-radiation cross-linking of poly(olefins), sulfur cross-linking of rubbers, e-beam cross-linking of Nylon, *etc.*), the incorporation of polymers or other materials into the resistors to enhance physical properties (for instance, the incorporation of a high molecular weight, high transition metal (Tm) polymers), the incorporation of the resistor elements into supporting matrices such as clays or polymer networks (forming the resistor blends within poly-(methylmethacrylate) networks or within the lamellae of montmorillonite, for instance), *etc*. In another embodiment, the resistor is deposited as a surface layer on a solid matrix which provides means for supporting the leads. Typically, the matrix is a chemically inert, non-conductive substrate such as a glass or ceramic.

Sensor arrays particularly well-suited to scaled up production are fabricated using integrated circuit (IC) design technologies. For example, the chemiresistors can easily be integrated onto the front end of a simple amplifier interfaced to an A/D converter to efficiently feed the data stream directly into a neural network software or hardware analysis section. Micro-fabrication techniques can integrate the chemiresistors directly onto a micro-chip which contains the circuitry for analogue signal conditioning/processing and then data analysis. This provides for the production of millions of incrementally different sensor elements in a single manufacturing step using ink-jet technology. Controlled compositional gradients in the chemiresistor elements of a sensor array can be induced in a method analogous to how a color ink-jet printer deposits and mixes multiple colors. However, in this case rather than multiple colors, a plurality of different polymers in solution which can be deposited are used. A sensor array of a million distinct elements only requires a 1 cm x 1 cm sized chip employing lithography at the 10 μm feature level, which is within the capacity of conventional commercial processing and deposition methods. This technology permits the production of sensitive, small-sized, stand-alone chemical sensors.

Preferred sensor arrays have a predetermined inter-sensor variation in the structure or composition of the nonconductive organic polymer regions. The variation may be quantitative and/or qualitative. For example, the concentration of the nonconductive organic polymer in the blend can be varied across sensors. Alternatively, a variety of different organic polymers may be used in different sensors. An electronic nose for detecting an analyte in a fluid is fabricated by electrically coupling the sensor leads of an array of compositionally different sensors to an electrical measuring device. The device measures changes in resistivity at each sensor of the array, preferably simultaneously and preferably over time. Frequently, the device includes signal processing means and is used in conjunction with a computer and data structure for comparing a given response profile to a structure-response profile database for qualitative and quantitative analysis. Typically such a nose comprises at least ten, usually at least 100, and often at least 1000 different sensors though with mass deposition fabrication techniques described herein or otherwise known in the art, arrays of on the order of at least $10^6$ sensors are readily produced.

In operation, each resistor provides a first electrical resistance between its conductive leads when the resistor is contacted with a first fluid comprising a chemical analyte at a first concentration, and a second electrical resistance between its conductive leads when the resistor is contacted with a second fluid comprising the same chemical analyte at a second different concentration. The fluids may be liquid or gaseous in nature. The first and second fluids may reflect samples from two different environments, a change in the concentration of an analvte in a fluid sampled at two time points, a sample and a negative control, *etc.* The sensor array necessarily comprises sensors which respond differently to a change in an analyte concentration, *i.e.*, the difference between the first and second electrical resistance of one sensor is different from the difference between the first second electrical resistance of another sensor.

In a preferred embodiment, the temporal response of each sensor (resistance as a function of time) is recorded. The temporal response of each sensor may be normalized to a maximum percent increase and percent decrease in resistance which produces a response pattern associated with the exposure of the analyte. By iterative profiling of known analytes, a structure-function database correlating analytes and response profiles is generated. Unknown analyte may then be characterized or identified using response pattern comparison and recognition algorithms. Accordingly, analyte detection systems comprising sensor arrays, an electrical measuring devise for detecting resistance across each chemiresistor, a computer, a data structure of sensor array response profiles, and a comparison algorithm are provided. In another embodiment, the electrical measuring device is an integrated cicuit comprising neural network-based hardware and a digital-analog converter (DAC) multiplexed to each sensor, or a plurality of DACs, each connected to different sensor(s).

A wide variety of analytes and fluids may be analyzed by the disclosed sensors, arrays and noses so long as the subject analyte is capable generating a differential response across a plurality of sensors of the array. Analyte applications include broad ranges of chemical classes such as organics such as alkanes, alkenes, alkynes, dienes, alicyclic hydrocarbons, arenes, alcohols, ethers, ketones, aldehydes, carbonyls, carbanions, polynuclear aromatics and derivatives of such organics, *e.g.*, halide derivatives, *etc.*, biomolecules such as sugars, isoprenes and isoprenoids, fatty acids and derivatives, *etc.* Accordingly, commercial applications of the sensors, arrays and noses include environmental toxicology and remediation, biomedicine, materials quality control, food and agricultural products monitoring, *etc.*

The general method for using the disclosed sensors, arrays and electronic noses, for detecting the presence of an analyte in a fluid involves resistively sensing the presence of an analyte in a fluid with a chemical sensor comprising first and second conductive leads electrically coupled to and separated by a chemically sensitive resistor as described above by measuring a first resistance between the conductive leads when the resistor is contacted with a first fluid comprising an analyte at a first concentration and a second different resistance when the resistor is contacted with a second fluid comprising the analyte at a second different concentration.

The following examples are offered by way of illustration and not by way of limitation.

Polymer Synthesis. Poly(pyrrole) films used for conductivity, electrochemical, and optical measurements were prepared by injecting equal volumes of $N_2$-purged solutions of pyrrole (1.50 mmoles in 4.0 ml dry tetrahydrofuran) and phosphomolybdic acid (0.75 mmoles in 4.0 ml tetrahydrofuran) into a $N_2$-purged test tube. Once the two solutions were mixed, the yellow phosphomolybdic acid solution turned dark green, with no observable precipitation for several hours. This solution was used for film preparation within an hour of mixing.

Instrumentation. Optical spectra were obtained on a Hewlett Packard 8452A spectrophotometer, interfaced to an IBM XT. Electrochemical experiments were performed using a Princeton Applied Research Inc. 173 potentiostat/175 universal programmer. All electrochemical experiments were performed with a Pt flag auxiliary and a saturated calomel reference electrode (SCE). Spin-coating was performed on a Headway Research Inc. photoresist spin coater. Film thicknesses were determined with a Dektak Model 3030 profilometer. Conductivity measurements were performed with an osmium-tipped four point probe (Alessi Instruments Inc., tip spacing = (0.050"), tip radii = (0.010")). Transient resistance measurements were made with a conventional multimeter (Fluke Inc., "Hydra Data Logger" Meter).

Principle Component Analysis and Multi-linear Least Square Fits. A data set obtained from a single exposure of the array to an odorant produced a set of descriptors (*i.e.*, resistances), $d_i$. The data obtained from multiple exposures thus produced a data matrix D where each row, designated by j, consisted of n descriptors describing a single member of the data set (*i.e.*, a single exposure to an odor). Since the baseline resistance and the relative changes in resistance varied among sensors, the data matrix was autoscaled before further processing (Hecht (1990) Mathematics in Chemistry: An Introduction to Modern Methods (Prentice Hall, Englewood Cliffs, NJ)). In this preprocessing technique, all the data associated with a single descriptor (*i.e.*, a column in the data matrix) were centered around zero with unit standard deviation

$$(1) \qquad d'_{ij} = (d_{ij} - \bar{d}_i)/\sigma_i$$

where $\bar{d}_i$ is the mean value for descriptor i and $\sigma_i$ is the corresponding standard deviation.

Principle component analysis (Hecht (1990) Mathematics in Chemistry: An Introduction to Modern Methods (Prentice Hall, Englewood Cliffs, NJ)) was performed to determine linear combinations of the data such that the maximum variance [defined as the square of the standard deviation] between the members of the data set was obtained in n mutually orthogonal dimensions. The linear combinations of the data resulted in the largest variance [or separation] between the members of the data set in the first principle component (pc1) and produced decreasing magnitudes of variance from the second to the $n^{th}$ principle component (pc2 - pcn). The coefficients required to transform the autoscaled data into principle component space (by linear combination) were determined by multiplying the data matrix, D, by its transpose, $D^T$ (*i.e.*, diagnolizing the matrix) (Hecht (1990) Mathematics in Chemistry: An Introduction to Modern Methods (Prentice Hall, Englewood Cliffs, NJ))

$$(2) \qquad R = D^T \cdot D$$

This operation produced the correlation matrix, R whose diagonal elements were unity and whose off-diagonal elements were the correlation coefficients of the data. The total variance in the data was thus given by the sum of the diagonal elements in R. The n eigenvalues, and the corresponding n eigenvectors, were then determined for R. Each eigenvector contained a set of n coefficients which were used to transform the data by linear combination into one of its n principle components. The corresponding eigenvalue yielded the fraction of the total variance that was contained in that principle component. This operation produced a principle component matrix, P, which had the same dimensions as the original data matrix. Under these conditions, each row of the matrix P was still associated with a particular odor and each column was associated with a particular principle component.

Since the values in the principle component space had no physical meaning, it was useful to express the results of the principle component analysis in terms of physical parameters such as partial pressure and mole fraction. This was achieved via a multi-linear least square fit between the principle component values and the corresponding parameter of interest. A multi-linear least square fit resulted in a linear combination of the principle components which yielded the best fit to the corresponding parameter value. Fits were achieved by appending a column with each entry being unity to the principle component matrix P, with each row, j, corresponding to a different parameter value (*e.g.*, partial pressure), $v_j$, contained in vector V. The coefficients for the best multi-linear fit between the principle components and parameter of interest were obtained by the following matrix operation

$$(3) \qquad C = (P^T \cdot P)^{-1} \cdot P^T \cdot V$$

where C was a vector containing the coefficients for the linear combination.

A key to our ability to fabricate chemically diverse sensing elements was the preparation of processable, air stable films of electrically conducting organic polymers. This was achieved through the controlled chemical oxidation of pyrrole (PY) using phosphomolybdic acid ($H_3PMo_{12}O_{40}$) (Pope (1983) Heteropoly and Isopoly Oxometalates (Springer-Verlag, New York), chap. 4) in tetrahydrofuran:

$$PY \rightarrow PY^{\bullet+} + e \qquad\qquad (4)$$

$$2\,PY^{\bullet+} \rightarrow PY_2 + 2H^+ \qquad\qquad (5)$$

$$H_3PMo_{12}O_{40} + 2e^- + 2H^+ \rightarrow H_5PMo_{12}O_{40} \qquad (6)$$

The redox-driven or electrochemically-induced polymerization of pyrrole has been explored previously, but this process typically yields insoluble, intractable deposits of poly(pyrrole) as the product (Salmon *et al*. (1982) J. Polym. Sci., Polym. Lett. 20: 187-193). Our approach was to use low concentrations of the $H_3PMo_{12}O_{40}$ oxidant ($E° = +0.36$ V vs. SCE). (Pope (1983) Heteropoly and Isopoly Oxometalates (Springer-Verlag, New York), chap. 4). Since the electrochemical potential of $PY^+/PY$ is more positive ($E° = +1.30$ V vs. SCE) (Andrieux *et al*. (1990) J.Am.Chem.Soc. 112:2439-2440) than that of $H_3PMo_{12}O_{40}/H_5PMo_{12}O_{40}$, the equilibrium concentration of $PY^{+\cdot}$, and thus the rate of polymerization, was relatively low in dilute solutions (0.19 M PY, 0.09 M $H_3PMo_{12}O_{40}$). However, it has been shown that the oxidation potential of pyrrole oligomers decreases from +1.20 V to +0.55 to +0.26 V vs. SCE as the number of units increase from one to two to three, and that the oxidation potential of bulk poly(pyrrole) occurs at -0.10 V vs. SCE (Diaz *et al*. (1981) J.Electroanal.Chem. 121:355-361). As a result, oxidation of pyrrole trimers by phosphomolybdic acid is expected to be thermodynamically favorable. This allowed processing of the monomer-oxidant solution (*i.e.*, spin coating, dip coating, introduction of plasticizers, *etc*.), after which time polymerization to form thin films was simply effected by evaporation of the solvent. The dc electrical conductivity of poly(pyrrole) films formed by this method on glass slides, after rinsing the films with methanol to remove excess phosphomolybdic acid and/or monomer, was on the order of 15 - 30 S-cm$^{-1}$ for films ranging from 40 - 100 nm in thickness.

The poly(pyrrole) films produced in this work exhibited excellent electrochemical and optical properties. For example, Fig. 42 shows the cyclic voltammetric behavior of a chemically polymerized poly(pyrrole) film following ten cycles from -1.00 V to +0.70 V vs. SCE. The cathodic wave at -0.40 V corresponded to the reduction of poly(pyrrole) to its neutral, nonconducting state, and the anodic wave at -0.20 V corresponded to the reoxidation of poly(pyrrole) to its conducting state (Kanazawa *et al*. (1981) Synth.Met. 4:119-130). The lack of additional faradaic current, which would result from the oxidation and reduction of phosphomolybdic acid in the film, suggests that the Keggin structure of phosphomolybdic acid was not present in the film anions (Bidan *et al*: (1988) J.Electroanal.Chem. 251:297-306) and implies that $MoO_4^{2-}$, or other anions, served as the poly(pyrrole) counterions in the polymerized films.

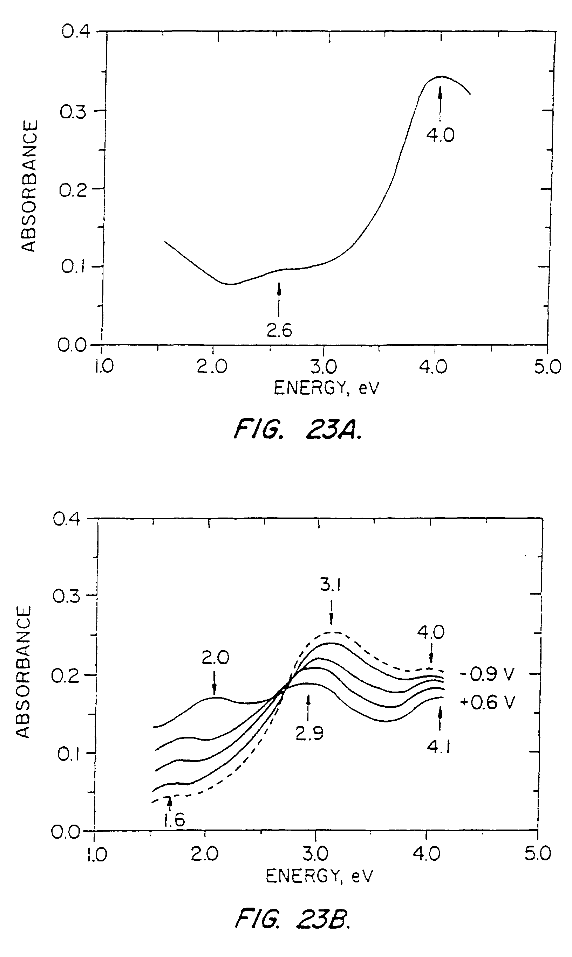

Fig 23A shows the optical spectrum of a processed polypyrrole film that had been spin-coated on glass and then rinsed with methanol. The single absorption maximum was characteristic of a highly oxidized poly(pyrrole) (Kaufman *et al*. (1984) Phys.Rev.Lett. 53:1005-1008), and the absorption band at 4.0 eV was characteristic of an interband transition between the conduction and valence bands. The lack of other bands in this energy range was evidence for the presence of bipolaron states (see Fig. 23A), as have been observed in highly oxidized poly(pyrrole) (Kaufman *et al*. (1984) Phys.Rev.Lett. 53:1005-1008). By cycling the film in 0.10 M $[(C_4H_9)_4N]^-[ClO_4]^-$ - acetonitrile and then recording the optical spectra in 0.10 M KCl - $H_2O$, it was possible to observe optical transitions characteristic of polaron states in oxidized poly(pyrrole) (see Fig. 23B). The polaron states have been reported to produce three optical transitions (Kaufman *et al.* (1984) Phys.Rev.Lett. 53:1005-1008), which were observed at 2.0, 2.9, and 4.1 eV in Fig. 23B. Upon reduction of the film (c.f. Fig 23B), an increased intensity and a blue shift in the 2.9 eV band was observed, as expected for the $\pi \rightarrow \pi^*$ transition associated with the pyrrole units contained in the polymer backbone (Yakushi *et al*. (1983) J.Chem. Phys. 79:4774-4778).

As described in the experimental section, various plasticizers were introduced into the polymer films (Table 3).

Table 3.

| Plasticizers used in array elements* | |
|---|---|
| Sensor | Plasticizer |
| 1 | None |
| 2 | none** |
| 3 | poly(styrene) |
| 4 | poly(styrene) |
| 5 | poly(styrene) |
| 6 | poly(a-methyl styrene) |
| 7 | poly(styrene-acrylonitrile) |
| 8 | poly(styrene-maleic anydride) |
| 9 | poly(styrene-allyl alcohol) |

*Sensors contained 2:3 (w:w) ratio of pyrrole to plasticizer.

**Film not rinsed to remove excess phosphomolybdic acid.

Table 3.   (continued)

| Plasticizers used in array elements* | |
|---|---|
| Sensor | Plasticizer |
| 10 | poly(vinyl pyrrolidone) |
| 11 | poly(vinyl phenol) |
| 12 | poly(vinyl butral) |
| 13 | poly(vinyl acetate) |
| 14 | poly(carbonate) |

*Sensors contained 2:3 (w:w) ratio of pyrrole to plasticizer.

These inclusions allowed chemical control over the binding properties and electrical conductivity of the resulting plasticized polymers. Sensor arrays consisted of as many as 14 different elements, with each element synthesized to produce a distinct chemical composition, and thus a distinct sensor response, for its polymer film. The resistance, R, of each film-coated individual sensor was automatically recorded before, during, and after exposure to various odorants. A typical trial consisted of a 60 sec rest period in which the sensors were exposed to flowing air (3.0 liter-min$^{-1}$), a 60 sec exposure to a mixture of air (3.0 liter-min$^{-1}$) and air that had been saturated with solvent (0.5 - 3.5 liter-min$^{-1}$), and then a 240 sec exposure to air (3.0 liter-min$^{-1}$).

In an initial processing of the data, presented in this paper, the only information used was the maximum amplitude of the resistance change divided by the initial resistance, $\Delta R_{max}/R_i$, of each individual sensor element. Most of the sensors exhibited either increases or decreases in resistance upon exposure to different vapors, as expected from changes in the polymer properties upon exposure to different types chemicals (Topart and Josowicz (1992) J. Phys.Chem. 96:7824-7830; and Charlesworth *et al*. (1993) J.Phys.Chem. 97:5418-5423). However, in some cases, sensors displayed an initial decrease followed by an increase in resistance in response to a test odor. Since the resistance of each sensor could increase and/or decrease relative to its initial value, two values of $\Delta R_{max}/R_i$ were reported for each sensor. The source of the bi-directional behavior of some sensor/odor pairs has not yet been studied in detail, but in most cases this behavior arose from the presence of water (which by itself induced rapid decreases in the film resistance) in the reagent-grade solvents used to generate the test odors of this study. The observed behavior in response to these air-exposed, water-containing test solvents was reproducible and reversible on a given sensor array, and the environment was representative of many practical odor sensing applications in which air and water would not be readily excluded.

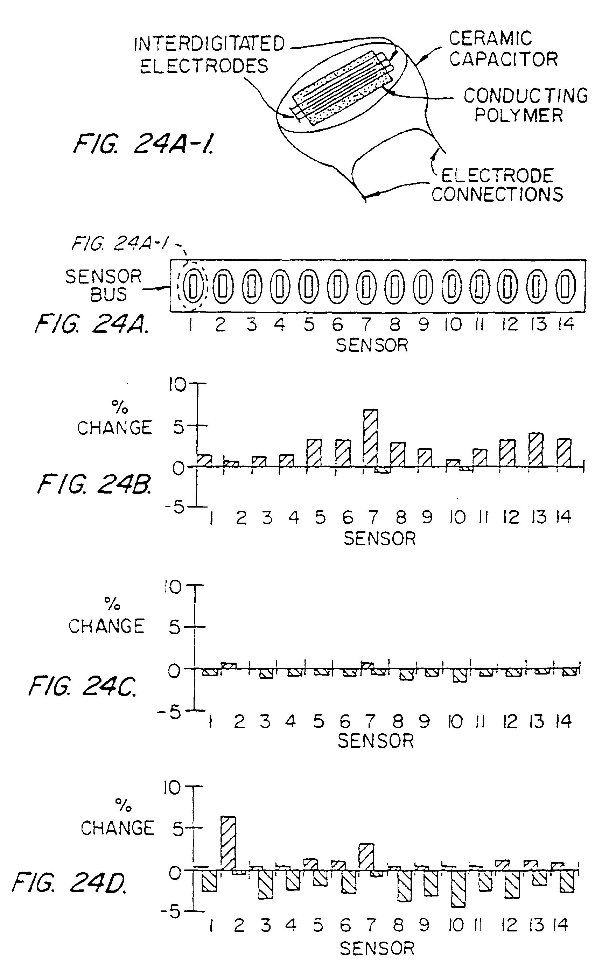

Figs. 24B-24D depict representative examples of sensor amplitude responses of a sensor array (see, Table 3). In this experiment, data were recorded for three separate exposures to vapors of acetone, benzene, and ethanol flowing in air. The response patterns generated by the sensor array described in Table 3 are displayed for: (B) acetone; (C) benzene; and (D) ethanol. The sensor response was defined as the maximum percent increase and decrease of the resistance divided by the initial resistance (gray bar and black bar, respectively) of each sensor upon exposure to solvent vapor. In many cases sensors exhibited reproducible increases and decreases in resistance. An exposure consisted of: (i) a 60 sec rest period in which the sensors were exposed to flowing air (3.0 liter-min$^{-1}$); (ii) a 60 sec exposure to a mixture of air (3.0 liter-min$^{-1}$) and air that had been saturated with solvent (0.5 liter-min$^{-1}$); and (iii) a 240 sec exposure to air (3.0 liter-min$^{-1}$). It is readily apparent that these odorants each produced a distinctive response on the sensor array. In additional experiments, a total of 8 separate vapors (acetone, benzene, chloroform, ethanol, isopropyl alcohol, methanol, tetrahydrofuran, and ethyl acetate), chosen to span a range of chemical and physical characteristics, were evaluated over a five-day period on a 14-element sensor array (Table 3). As discussed below, each odorant could be clearly and reproducibly identified from the others using this sensor apparatus.

Principle component analysis (Hecht (1990) Mathematics in Chemistry: An Introduction to Modern Methods (Prentice Hall, Englewood Cliffs, NJ)) was used to simplify presentation of the data and to quantify the distinguishing abilities of individual sensors and of the array as a whole. In this approach, linear combinations of the $\Delta R_{max}/R_i$ data for the elements in the array were constructed such that the maximum variance (defined as the square of the standard deviation) was contained in the fewest mutually orthogonal dimensions. This allowed representation of most of the information contained in data sets shown in Figs. 24B-24D in two (or three) dimensions. The resulting clustering, or lack thereof, of like exposure data in the new dimensional space was used as a measure of the distinguishing ability, and of the reproducibility, of the sensor array.

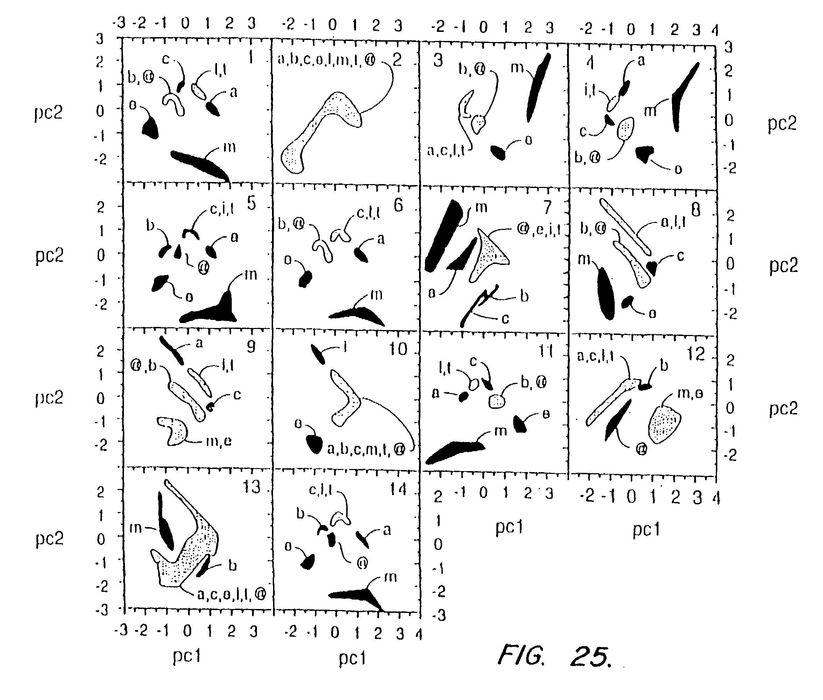

In order to illustrate the variation in sensor response of individual sensors that resulted from changes in the plasticizing polymer, principle component analysis was performed on the individual, isolated responses of each of the 14 individual sensor elements in a typical array (Fig. 25). Data were obtained from multiple exposures to acetone (a), benzene (b), chloroform (c), ethanol (e), isopropyl alcohol (i), methanol (m), tetrahydrofuran (t), or ethyl acetate (@) over a period of five days with the test vapors exposed to the array in various sequences. The numbers of the figures refer to the sensor elements described in Table 3. The units along the axes indicate the amplitude of the principle component that was used to describe the particular data set for an odor. The black regions indicate clusters corresponding to a single solvent which could be distinguished from all others; gray regions highlight data of solvents whose signals overlapped with others around it. Exposure conditions were identical to those in Fig. 24.

Since each individual sensor produced two data values, principle component analysis of these responses resulted in only two orthogonal principal components; pc1 and pc2. As an example of the selectivity exhibited by an individual sensor element, the sensor designated as number 5 in Fig. 25 (which was plasticized with poly(styrene)) confused acetone with chloroform, isopropyl alcohol, and tetrahydrofuran. It also confused benzene with ethyl acetate, while easily distinguishing ethanol and methanol from all other solvents. Changing the plasticizer to poly ($\alpha$-methyl styrene) (sensor number 6 in Fig. 25) had little effect on the spatial distribution of the responses with respect to one another and with respect to the origin. Thus, as expected, a rather slight chemical modification of the plasticizer had little effect on the relative variance of the eight test odorants. In contrast, the addition of a cyano group to the plasticizer, in the form of poly(styrene-acrylonitrile), (sensor number 7 in Fig. 25), resulted in a larger contribution to the overall variance by benzene and chloroform, while decreasing the contribution of ethanol. Changing the substituent group in the plasticizer to a hydrogen bonding acid (poly(styrene-allyl alcohol), sensor number 9 in Fig. 25) increased the contribution of acetone to the overall variance while having little effect on the other odors, with the exception of confusing methanol and ethanol. These results suggest that the behavior of the sensors can be systematically altered by varying the chemical composition of the plasticizing polymer.

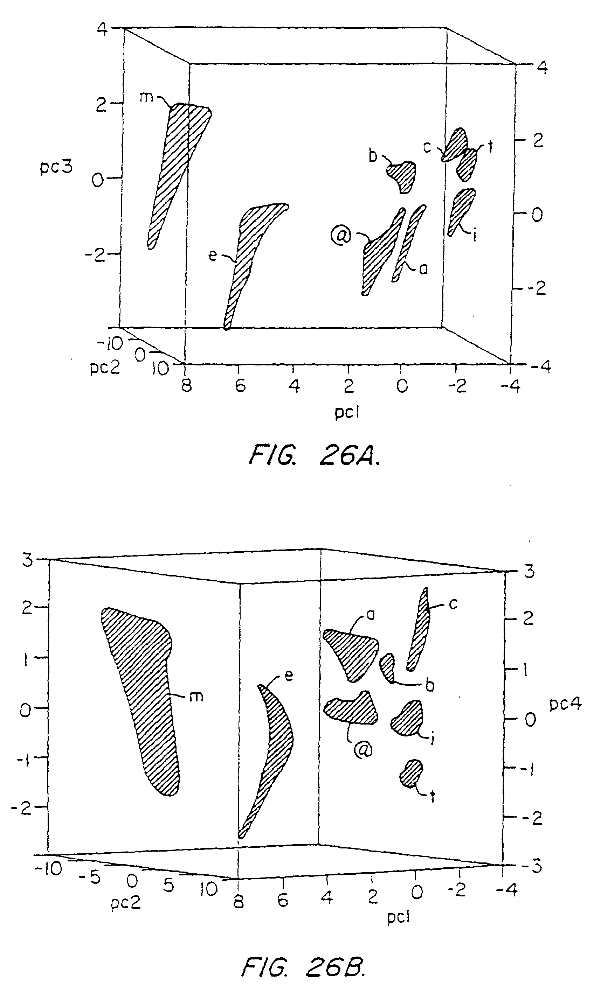

Fig. 26 shows the principle component analysis for all 14 sensors described in Table 3 and Figs. 24 and 25. When the solvents were projected into a three dimensional odor space (Fig. 26A or 26B), all eight solvents were easily distinguished with the specific array discussed herein. Detection of an individual test odor, based only on the criterion of observing -1% $\Delta R_{max}/R_i$ values for all elements in the array, was readily accomplished at the parts per thousand level with no control over the temperature or humidity of the flowing air. Further increases in sensitivity are likely after a thorough utilization of the temporal components of the $\Delta R_{max}/R_i$ data as well as a more complete characterization of the noise in the array.

We have also investigated the suitability of this sensor array for identifying the components of certain test mixtures. This task is greatly simplified if the array exhibits a predictable signal response as the concentration of a given odorant is varied, and if the responses of various individual odors are additive (*i.e.*, if superposition is maintained). When a 19-element sensor array was exposed to a number, n, of different acetone concentrations in air, the $(CH_3)_2CO$ concentration was semi-quantitatively predicted from the first principle component. This was evident from a good linear least square fit through the first three principle components.

The same sensor array was also able to resolve the components in various test methanol-ethanol mixtures (Morris *et al.* (1942) Can.J.Res. B20:207-211). As shown in Fig. 278, a linear relationship was observed between the first principle component and the mole fraction of methanol in the liquid phase, $x_m$, in a $CH_3OH$-$C_2H_5OH$ mixture, demonstrating that superposition held for this mixture/sensor array combination. Furthermore, although the components in the mixture could be predicted fairly accurately from just the first principle component, an increase in the accuracy could be achieved using a multi-linear least square fit through the first three principle components. This relationship held for $CH_3OH/(CH_3OH + C_2H_5OH)$ ratios of 0 to 1.0 in air-saturated solutions of this vapor mixture. The conducting polymer-based sensor arrays could therefore not only distinguish between pure test vapors, but also allowed analysis of concentrations of odorants as well as analysis of binary mixtures of vapors.

In summary, the results presented herein advance the area of analyte sensor design. A relatively simple array design, using only a multiplexed low-power dc electrical resistance readout signal, has been shown to readily distinguish between various test odorants. Such conducting polymer-based arrays are simple to construct and modify, and afford an opportunity to effect chemical control over the response pattern of a vapor. For example, by increasing the ratio of plasticizer to conducting polymer, it is possible to approach the percolation threshold, at which point the conductivity exhibits a very sensitive response to the presence of the sorbed molecules. Furthermore, producing thinner films will afford the opportunity to obtain decreased response times, and increasing the number of plasticizing polymers and polymer backbone motifs will likely result in increased diversity among sensors. This type of polymer-based array is chemically flexible, is simple to fabricate, modify, and analyze, and utilizes a low power dc resistance readout signal transduction path to convert chemical data into electrical signals. It provides a new approach to broadly-responsive odor sensors for fundamental and applied investigations of chemical mimics for the mammalian sense of smell. Such systems are useful for evaluating the generality of neural network algorithms developed to understand how the mammalian olfactory system identifies the directionality, concentration, and identity of various odors.

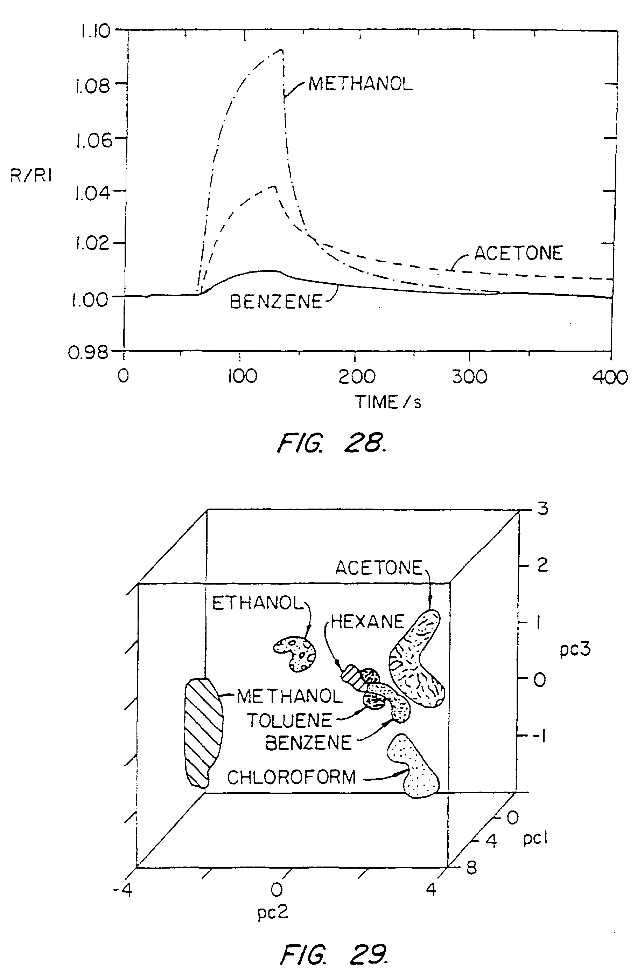

Sensor Array Testing. To evaluate the performance of the carbon-black based sensors, arrays with as many as 20 elements were exposed to a series of analytes. A sensor exposure consisted of (1) a 60 second exposure to flowing air (6 liter min-1), (2) a 60 second exposure to a mixture of air (6 liter min-1) and air that had been saturated with the analyte (0.5 liter min-1), (3) a five minute recovery period during which the sensor array was exposed to flowing air (6 liter min-1). The resistance of the elements were monitored during exposure, and depending on the thickness and chemical make-up of the film, resistance changes as large as 250% could be observed in response to an analyte. In one experiment, a 10 element sensor array consisting carbon-black composites formed with a series of non-conductive polymers (see Table 4) was exposed to acetone, benzene, chloroform, ethanol, hexane, methanol, and toluene over a two day period. A total of 58 exposures to these analytes were performed in this time period. In all cases, resistance changes in response to the analytes were positive, and with the exception of acetone, reversible (see Figure 28). The maximum positive deviations were then subjected to principal component analysis in a manner analogous to that described for the poly(pyrrole) based sensor. Figure 29 shows the results of the principal component analysis for the entire 10-element array. With the exception of overlap between toluene with benzene, the analytes were distinguished from one and other.

The apparatuses can include a chemical sensor comprising first and second conductive elements (*e.g.*, electrical leads) capacitively coupled to a chemically sensitive element. Variability in chemical sensitivity from sensor to sensor is conveniently provided by qualitatively or quantitatively varying the composition of the sensor film. For example, in one embodiment, a nonconductive organic polymer varies between sensors (*e.g.*, different plastics such as polystyrene).

Arrays of such sensors can be constructed with at least two sensors having different chemically sensitive elements providing dissimilar differences in capacitance, impedance or inductance. An electronic nose for detecting an analyte in a fluid may be constructed by using such arrays in conjunction with an electrical measuring device electrically connected to the elements of each sensor. Such electronic noses may incorporate a variety of additional components including means for monitoring the temporal response of each sensor, assembling and analyzing sensor data to determine analyte identity, etc. Methods of making and using the disclosed sensors, arrays and electronic noses are also described herein.

Sensor arrays for detecting an analyte in a fluid for use in conjunction with an electrical measuring apparatus comprise a plurality of compositionally different chemical sensors. Each sensor comprises a chemically sensitive capacitive, impedance or inductance element. The leads may be any convenient conductive material, usually a metal, and may be interdigitized or otherwise configured to maximize signal-to-noise strength.

A wide variety of materials can be used for the capacitive, impedance or inductive elements. Table 1 provides some exemplary organic polymers. Blends and copolymers, such as of the polymers listed above in Table 2, may also be used. In addition to the exemplary organic polymers, other dielectric or thin films of inorganic materials can be used (*e.g.*; mica). Still further, combinations of organic polymers and dielectric inorganic materials are useful (*e.g.*, blends of poly(methacrylates) and mica). Combinations, concentrations, blend stoichiometries, etc. are readily determined empirically by fabricating and screening prototype capacitive, impedance or inductive elements as described below.

The capacitive, impedance or inductive elements can be fabricated by many techniques such as, but not limited to, solution casting, suspension casting, and mechanical mixing. In general, solution cast routes are advantageous because they provide homogeneous structures and ease of processing. With solution cast routes, capacitive, impedance or inductive elements may be easily fabricated by spin, spray or dip coating. Suspension casting provides the possibility of spin, spray or dip coating but more heterogeneous structures than with solution casting are expected. With mechanical mixing, there are no solubility restrictions since it involves only the physical mixing of the sensor components, but device fabrication is more difficult since spin, spray and dip coating are no longer possible. A more detailed discussion of each of these follows.

For systems where both the capacitive, impedance or inductive elements or their reaction precursors are soluble in a common solvent, the elements can be fabricated by solution casting. The choice of materials in this route is, of course, limited to those that are soluble in the reaction media.

In suspension cashing, one or more of the components of the capacitive, impedance or inductive element is suspended and the others dissolved in a common solvent. Suspension casting is a rather general technique applicable to a wide range of species which can be suspended in solvents by vigorous mixing or sonication. In one application of suspension casting, a non-conductive polymer is dissolved in an appropriate solvent (such as THF, acetonitrile, water, *etc.*) and the resulting solution is used to dip coat electrodes.

Mechanical mixing is suitable for all of the sensor elements if additional dielectric materials are to be included in the formulations. In this technique, the materials are physically mixed in a ball-mill or other mixing device. For instance, mica : non-conductive polymer composites are readily made by ball-milling. When the non-conductive polymer can be melted or significantly softened without decomposition, mechanical mixing at elevated temperature can improve the mixing process. Alternatively, composite fabrication can sometimes be improved by several sequential heat and mix steps.

Once fabricated, the individual elements can be optimized for a particular application by varying their chemical make up and morphologies. The chemical nature of the capacitive, impedance or inductive elements determines to which analytes they will respond and their ability to distinguish different analytes. The film morphology is also important in determining response characteristics. For instance, thin films respond more quickly to analytes than do thick ones.

Hence, with an empirical catalogue of information on chemically diverse sensors made with different components and by differing fabrication routes, sensors can be chosen that are appropriate for the analytes expected in a particular application, their concentrations, and the desired response times. Further optimization can then be performed in an iterative fashion as feedback on the performance of an array under particular conditions becomes available.

The sensor arrays particularly are fabricated using integrated circuit (IC) design technologies. For example, the capacitive, impedance or inductive elements can easily be integrated with measurement circuitry interfaced to an A/D converter to efficiently feed the data stream directly into a neural network software or hardware analysis section. Micro-fabrication techniques can integrate the capacitive, impedance or inductive elements directly onto a micro-chip which contains the circuitry for analogue signal conditioning/processing and then data analysis. This provides for the production of millions of incrementally different sensor elements in a single manufacturing step using ink-jet technology. Controlled compositional gradients in the capacitive, impedance or inductive elements of a sensor array can be induced in a method analogous to how a color ink-jet printer deposits and mixes multiple colors. However, in this case rather than multiple colors, a plurality of different polymers (or compositions) in solution which can be deposited are used. As used herein, the term "different polymers" refers not only to chemically distinct polymers or compositions, but also to fixed compositions at a plurality of temperatures such that the signal produced by an individual sensor element is distinct and different from other sensor elements. Thus, an array of sensors could also comprise variation not in the composition of the material, but in external variables such as temperature and pressure.

The sensor arrays can comprise from 2 to 1,000,000 individual capacitive, impedance or inductive elements, each of which is a distinct composition of one or more of the following: non-conducting organic polymer, organic oligomer, other insulating dielectric materials or dielectric inorganic materials. For example, a sensor element can be a combination of two or more organic polymers, preferably of from two to twenty polymers. In addition to the methods above for the deposition of capacitive, impedance or inductive elements onto a substrate, combinatorial methods may also be used for generating the sensor arrays. In one such method, the above deposition techniques are used for the deposition of from 1 to 50 monomers in a combinatorial manner on a solid support. Suitable monomers include the single unit constituent materials for the polymers provided in Table 1. The monomeric depositions may also include dielectric inorganic materials (*e.g.*, mica). Following deposition, the monomeric mixtures can be polymerized using heat, light or other polymerization techniques known to those of skill in the art. In this manner, arrays of materials including polymers containing dielectric inorganic materials are produced. Using the described deposition techniques and either monomeric or polymeric components, sensor arrays having up to about 1,000,000 capacitive, impedance or inductive elements can be produced. In preferred embodiments, the array will have from 9 (a $3 \times 3$ matrix) to 10,000 (a $100 \times 100$ matrix) capacitive, impedance or inductive elements. In some embodiments, arrays having from 49 to about 900 capacitive, impedance or inductive elements will be preferred.

A sensor array of 1,000,000 distinct elements only requires a 1 cm x 1 cm sized chip employing lithography at the 10 μm feature level, which is within the capacity of conventional commercial processing and deposition methods. This technology permits the production of sensitive, small-sized, stand-alone chemical sensors.

Preferred sensor arrays have a predetermined inter-sensor variation in the structure or composition of the sensor elements. The variation may be quantitative and/or qualitative. For example, the concentration of a nonconductive organic polymer in a blend can be varied across sensors. Alternatively, a variety of different organic polymers may be used in different sensors. An electronic nose for detecting an analyte in a fluid is fabricated by electrically coupling the sensor leads of an array of compositionally different sensors to an electrical measuring device. The device measures changes in impedance at each sensor of the array, preferably simultaneously and preferably over time. Frequently, the device includes signal processing means and is used in conjunction with a computer and data structure for comparing a given response profile to a structure-response profile database for qualitative and quantitative analysis. Typically such a nose comprises at least ten, usually at least 100, and often at least 1000 different sensors though with mass deposition fabrication techniques described herein or otherwise known in the art, arrays of on the order of at least $10^6$ sensors are readily produced.

In operation, each capacitive, impedance or inductive element provides a first electrical signal between its conductive leads when the capacitive, impedance or inductive element is contacted with a first fluid comprising a chemical analyte at a first concentration, and a second signal between its conductive leads when the capacitive, impedance or inductive element is contacted with a second fluid comprising the same chemical analyte at a second different concentration. The fluids may be liquid or gaseous in nature. The first and second fluids may reflect samples from two different environments, a change in the concentration of an analyte in a fluid sampled at two time points, a sample and a negative control, *etc.* The sensor array necessarily comprises sensors which respond differently to a change in an analyte concentration, *i.e.*, the difference between the first and second signal of one sensor is different from the difference between the first second signal of another sensor.

In a preferred embodiment, the temporal response of each sensor (impedance as a function of time) is recorded. The temporal response of each sensor may be normalized to a maximum percent increase and percent decrease in impedance which produces a response pattern associated with the exposure of the analyte. By iterative profiling of known analytes, a structure-function database correlating analytes and response profiles is generated. Unknown analyte may then be characterized or identified using response pattern comparison and recognition algorithms. Accordingly, analyte detection systems comprising sensor arrays, an electrical measuring devise for detecting impedance or inductance across each capacitive, impedance or inductive element, a computer, a data structure of sensor array response profiles, and a comparison algorithm are provided. In another embodiment, the electrical measuring device is an integrated circuit comprising neural network-based hardware and a digital-analog converter (DAC) multiplexed to each sensor, or a plurality of DACs, each connected to different sensor(s).

A wide variety of analyses and fluids may be analyzed by the disclosed sensors, arrays and noses so long as the subject analyte is capable generating a differential response across a plurality of sensors of the array. Analyte applications include broad ranges of chemical classes such as organics such as alkanes, alkenes, alkynes, dienes, alicyclic hydrocarbons, arenes, alcohols, ethers, ketones, aldehydes, carbonyls, carbanions, polynuclear aromatics and derivatives of such organics, *e.g.*, halide derivatives, *etc.*, biomolecules such as sugars, isoprenes and isoprenoids, fatty acids and derivatives, *etc.* Accordingly, commercial applications of the sensors, arrays and noses include environmental toxicology and remediation, biomedicine, materials quality control, food and agricultural products monitoring, *etc.*

The general method for using the disclosed sensors, arrays and electronic noses, for detecting the presence of an analyte in a fluid involves capacitively, resistively or inductively sensing the presence of an analyte in a fluid with a chemical sensor comprising first and second conductive leads electrically coupled to and separated by a chemically sensitive capacitive, impedance or inductive element as described above by measuring a first impedance or inductance between the conductive leads when the capacitive, impedance or inductive element is contacted with a first fluid comprising an analyte at a first concentration and a second different impedance or inductance when the capacitive, impedance or inductive element is contacted with a second fluid comprising the analyte at a second different concentration.

The foregoing description of preferred embodiments of the invention has been presented for the purposes of illustration and description. It is not intended to be exhaustive or to limit the invention to the precise form described, and many modifications and variations are possible in light of the teaching above. The embodiments were chosen and described in order to best explain the principles of the invention and its practical applications to thereby enable others skilled in the art to best utilize and practice the invention in various embodimencs and with various modifications as are suited to the particular use contemplated. It is intended that the scope of the invention be defined by the following claims.

### Claims

1. An integrated circuit (205) comprising:

   a plurality of sensor sites (210) formed on the integrated circuit, wherein a sensor material (330) is constrained at the sensor site and the sensor material (330) has regions of a nonconductive organic material and a conductive material, and in the presence of an analyte, the sensor material having measurable changes in an electrical property; and
   electrical terminals (710, 715) formed to couple to the sensor material at the sensor sites (310), wherein the electrical terminals transmit electrical signals to evaluate the change in the electrical property of the sensor material; **characterised by**:

   the sensor site comprising a sensor hurdle.

2. An integrated circuit (205) as claimed in Claim 1, wherein the electrical property comprises resistance.

3. An integrated circuit (205) as claimed in Claim 1, wherein the electrical property comprises capacitance.

4. An integrated circuit (205) as claimed in Claim 1, wherein the electrical property comprises inductance.

5. An integrated circuit (205) as claimed in Claim 1, wherein the nonconductive organic material is a polymer.

6. An integrated circuit (205) as claimed in Claim 1, wherein the conductive material is selected from the group consisting of carbon black, conducting organic conductors, metals, metal colloids, and inorganic conductors.

7. An electronic system for olfaction comprising an integrated circuit (205) as recited in Claim 1.

8. An integrated circuit (205) as claimed in Claim 1, wherein the sensor material (330) at a first sensor site has a different composition from the sensor material at a second sensor site.

9. An integrated circuit (205) as claimed in Claim 1, wherein the sensor material (330) at one sensor site is different from the sensor material (330) at other sensor sites.

10. An integrated circuit (205) as claimed in Claim 1, further comprising:

   a plurality of transistors (720, 735, 740, 750) formed on the integrated circuit and coupled to the electrical terminals (710, 715).

11. An integrated circuit (205) as claimed in Claim 1, wherein the material is from about 0.1 micron to about 1 micron thick.

12. An integrated circuit (205) as claimed in Claim 1, wherein the sensor material (330) at one sensor site is separated from the sensor material at another sensor site by a separating material.

13. An integrated circuit (205) as claimed in Claim 12, wherein the separating material is formed using a high impedance film (915).

14. An integrated circuit (205) as claimed in Claim 1, further comprising:

   a plurality of electronic circuits (1630), each circuit associated with one of the plurality of sensors and coupled to the terminals (710, 715) of the associated sensor.

15. An integrated circuit (205) as claimed in Claim 1, wherein the sensor material (330) at a sensor site is electrically coupled to the sensor material at an adjacent sensor site.

16. An integrated circuit (205) as claimed in Claim 1, wherein the sensor material (330) at a sensor site is coupled to the sensor material at an adjacent sensor site through a resistive element.

17. An integrated circuit (205) as claimed in Claim 14, wherein each electronic circuit (1630) communicates to another electronic circuit.

18. An integrated circuit (205) as claimed in Claim 14, wherein the plurality of sensor sites (210) is arranged in an array form (330) of rows and columns, and the integrated circuit (205) further comprises:

   a row multiplexer to select a row in the array of sensor sites; and
   a column multiplexer to select a column in the array of sensor sites.

19. An integrated circuit as claimed in Claim 14, wherein the electronics (1630) generates a change signal to indicate changed data at a specific sensor site, and the integrated circuit further comprises:

   a multiplexer circuit to determine and select the location of the specific sensor site having changed data.


**Patentansprüche**

1. Ein integrierter Schaltkreis (205), mit:

   einer Vielzahl von Sensorstellen (210), die auf dem integrierten Schaltkreis gebildet sind, wobei ein Sensormaterial (33) an der Sensorstelle gehalten ist, und das Sensormaterial (33) Bereiche eines nichtleitfähigen organischen Materials und eines leitfähigen Materials hat, und in der Gegenwart eines Analyten das Sensormaterial messbare Änderungen in einer elektrischen Eigenschaft und
   elektrische Anschlüsse (710, 715) ausgebildet sind, um das Sensormaterial mit den Sensorstellen (310) zu koppeln, wobei die elektrischen Anschlüsse elektrische Signale übertragen, um den Wechsel in der elektrischen Eigenschaft des Sensormaterials auszuwerten;

**dadurch gekennzeichnet, dass**:

die Sensorstelle eine Sensorhürde aufweist.

**2.** Ein integrierter Schaltkreis (205) beansprucht wie in Anspruch 1, wobei die elektrische Eigenschaft den Widerstand umfasst.

**3.** Ein integrierter Schaltkreis (205) beansprucht wie in Anspruch 1, bei dem die elektrische Eigenschaft die Kapazität umfasst.

**4.** Ein integrierter Schaltkreis (205) beansprucht wie in Anspruch 1, bei dem die elektrische Eigenschaft die Induktivität umfasst.

**5.** Ein integrierter Schaltkreis (205) beansprucht wie in Anspruch 1, bei dem das nichtleitfähige organische Material ein Polymer ist.

**6.** Ein integrierter Schaltkreis (205) beansprucht wie in Anspruch 1, bei dem das leitfähige Material ausgewählt ist aus der Gruppe, die aus Kohlenschwarz, leitfähigem organischen Leitern, Metallen, Metallkolloiden, und anorganischen Leitern gebildet ist.

**7.** Ein elektronisches System für den Riechvorgang, aufweisend einen integrierten Schaltkreis (205) wie in Anspruch 1 beschrieben.

**8.** Ein integrierter Schaltkreis (205) beansprucht wie in Anspruch 1, bei dem das Sensormaterial (330) an einer ersten Sensorstelle eine andere Zusammensetzung als das Sensormaterial an einer zweiten Sensorstelle hat.

**9.** Ein integrierter Schaltkreis (205) beansprucht wie in Anspruch 1, bei dem das Sensormaterial (330) an einer Sensorstelle von dem Sensormaterial (330) an anderen Sensorstellen abweicht.

**10.** Ein integrierter Schaltkreis (205) beansprucht wie in Anspruch 1, des Weiteren aufweisend:

eine Vielzahl von Transistoren (720, 735, 740, 750) ausgebildet auf dem integrierten Schaltkreis und angeschlossen an die elektrischen Anschlüsse (710, 715).

**11.** Ein integrierter Schaltkreis (205) beansprucht wie in Anspruch 1, bei dem das Material zwischen 0,1 Mikrometer und etwa 1 Mikrometer dick ist.

**12.** Ein integrierter Schaltkreis (205) beansprucht wie in Anspruch 1, bei dem das Sensormaterial (330) an einer Sensorstelle von dem Sensormaterial an einer anderen Sensorstelle durch ein Trennmaterial getrennt ist.

**13.** Ein integrierter Schaltkreis (205) beansprucht wie in Anspruch 12, bei dem das Trennmaterial unter Verwendung eines Hochimpedanz-Films (915) gebildet ist.

**14.** Ein integrierter Schaltkreis (205) beansprucht wie in Anspruch 1, des Weiteren aufweisend:

eine Vielzahl von elektronischen Schaltkreisen (1630), wobei jeder Schaltkreis einem der Vielzahl von Sensoren zugeordnet ist und mit den Anschlüssen (710, 715) des zugeordneten Sensors gekoppelt ist.

**15.** Ein integrierter Schaltkreis (205) beansprucht wie in Anspruch 1, wobei das Sensormaterial (330) an einer Sensorstelle elektrisch gekoppelt ist mit dem Sensormaterial an einer benachbarten Sensorstelle.

**16.** Ein integrierter Schaltkreis (205) beansprucht wie in Anspruch 1, bei dem das Sensormaterial (330) an einer Sensorstelle durch ein Widerstandselement mit dem Sensormaterial an einer benachbarten Sensorstelle gekoppelt ist.

**17.** Ein integrierter Schaltkreis (205) beansprucht wie in Anspruch 14, bei dem jeder elektronische Schaltkreis (1630) mit einem anderen elektronischen Schaltkreis kommuniziert.

**18.** Ein integrierter Schaltkreis (205) beansprucht wie in Anspruch 14, bei dem die Vielzahl von Sensorstellen (210) in einer Matrixform (330) von Zeilen und Spalten angeordnet ist, und bei dem der integrierte Schaltkreis (205) des Weiteren aufweist:

einen Zeilenmultiplexer, um eine Zeile in der Matrix von Sensorstellen auszuwählen, und einen Spaltenmultiplexer, um eine Spalte in der Matrix von Sensorstellen auszuwählen.

19. Ein integrierter Schaltkreis (205) beansprucht wie in Anspruch 14, bei dem die Elektronik (1630) ein Wechselsignal erzeugt, um geänderte Daten an einer spezifischen Sensorstelle anzugeben, und der integrierte Schaltkreis des Weiteren aufweist:

einen Multiplexerschaltkreis, um den Ort der spezifischen Sensorstelle zu bestimmen und auszuwählen, welche Daten gewechselt hat.

**Revendications**

1. Circuit intégré (205) comprenant :

une pluralité de sites de capteurs (210) formés sur le circuit intégré, dans lequel un matériau de capteur (330) est retenu sur le site de capteur et le matériau de capteur (330) possède des régions formées d'un matériau organique non conducteur et d'un matériau conducteur, et en présence d'un analyte, le matériau de capteur présente des variations mesurables d'une propriété électrique; et
des bornes électriques (710,715) formées de manière à être couplées au matériau de capteur au niveau des sites de capteurs (310), lorsque les bornes électriques transmettent des signaux électriques pour évaluer la modification de la propriété électrique du matériau de capteur;

**caractérisé par**
le site de capteur comprend une barrière de capteur.

2. Circuit intégré (205) selon la revendication 1, dans lequel la propriété électrique comprend une résistance.

3. Circuit intégré (205) selon la revendication 1, dans lequel la propriété électrique comprend une capacité.

4. Circuit intégré (205) selon la revendication 1, dans lequel la propriété électrique comprend une inductance.

5. Circuit intégré (205) selon la revendication 1, dans lequel le matériau organique non conducteur est un polymère.

6. Circuit intégré (205) selon la revendication 1, dans lequel le matériau conducteur est choisi dans le groupe comprenant le noir de carbone, des conducteurs organiques de conduction, des métaux, des colloïdes métalliques et des conducteurs minéraux.

7. Système électronique pour une détection olfactive comprenant un circuit intégré (205) selon la revendication 1.

8. Circuit intégré (205) selon la revendication 1, dans lequel le matériau de capteur (330) au niveau d'un premier site de capteur possède une composition différente du matériau de capteur en un second site de capteur.

9. Circuit intégré (205) selon la revendication 1, dans lequel le matériau de capteur (330) sur un site de capteur diffère du matériau de capteur (330) en d'autres sites de capteurs.

10. Circuit intégré (205) selon la revendication 1, comprenant en outre :

une pluralité de transistors (720,735,740,750) formés sur le circuit intégré et couplés aux bornes électriques (710,715).

11. Circuit intégré (205) selon la revendication 1, dans lequel le matériau possède une épaisseur entre environ 0,1 micromètre et environ 1 micromètre.

12. Circuit intégré (205) selon la revendication 1, dans lequel le matériau de capteur (330) au niveau d'un site de capteur est séparé du matériau de capteur en un autre site de capteur par un matériau de séparation.

13. Circuit intégré (205) selon la revendication 12, dans lequel le matériau de séparation est formé en utilisant un film à impédance élevée (915).

14. Circuit intégré (205) selon la revendication 1, comprenant en outre :

    une pluralité de circuits électroniques (1630), chaque circuit étant associé à l'un de la pluralité de capteurs et couplé aux bornes (710,715) du capteur associé.

15. Circuit intégré (205) selon la revendication 1, dans lequel le matériau de capteur (330) sur un site de capteur est couplé électriquement au matériau de capteur en un site de capteur adjacent.

16. Circuit intégré (205) selon la revendication 1, dans lequel le matériau de capteur (330) en un site de capteur est couplé au matériau de capteur en un site de capteur adjacent au moyen d'un élément résistif.

17. Circuit intégré (205) selon la revendication 14, dans lequel chaque circuit électronique (1630) communique avec un autre circuit électronique.

18. Circuit intégré (205) selon la revendication 14, dans lequel la pluralité de sites de capteurs (210) est agencée sous la forme d'une matrice (330) de lignes et de colonnes, et le circuit intégré (205) comprend en outre :

    un multiplexeur de lignes pour sélectionner une ligne dans le réseau de sites de capteurs; et
    un multiplexeur de colonnes pour sélectionner une colonne dans le réseau de sites de capteurs.

19. Circuit intégré (205) selon la revendication 14, dans lequel le système électronique (1630) génère un signal de changement pour indiquer des données modifiées en un site de capteur spécifique, et le circuit intégré comprend en outre :

    un circuit multiplexeur pour déterminer et sélectionner l'emplacement du site de capteur spécifique ayant des données modifiées.

*FIG. 1*

*FIG. 2A*

CHEMIAL SENSOR
(FILLING WELL)

SIGNAL PROCESSING
(ON-CHIP CIRCUITRY)

SILICON WAFER

*FIG. 2B*

FIG. 2C

330

SENSOR ARRAY

POLYMER A
POLYMER B
POLYMER C
POLYMER D
POLYMER E
POLYMER F
POLYMER G
POLYMER H
POLYMER I

$\Delta R/R$

ODOR A

ODOR B

AMPLITUDE

ELEMENT #

340

AMPLITUDE

ELEMENT #

350

*FIG. 3*

FIG. 4

FIG. 5

FIG. 6

EP 1 019 715 B1

FIG. 7

FIG. 8A

FIG. 8B

FIG. 8C

FIG. 8D

FIG. 8E

FIG. 8F

FIG. 9

FIG. 10

FIG. 11

FIG. 12

FIG. 13

FIG. 14

FIG. 15

UNIT CELL

SENSOR

ELECTRONICS

OUTPUT

1640

1630

1620

1650

1610

1660

1670

*FIG. 16*

$i_{BIAS}$

ROW ADDRESS MUX

ROW ADDRESS

COLUMN ADDRESS MUX

$R_{LOAD}$

A/D

CPU

*FIG. 17*

FIG. 18

FIG. 19

FIG. 20A.

FIG. 20B.

CONDUCTING
POLYMER,
PLASTICIZER

INTERDIGITATED
ELECTRODE
ARRAY

*FIG. 21A-1.*

FIG. 21A-1

ELEMENT, CONDUCTING POLYMER, PLASTICIZER

1, POLY(PYRROLE), NONE
2, POLY(PYRROLE), NONE
3, POLY(PYRROLE), POLY(STYRENE)
4, POLY(PYRROLE), POLY(STYRENE)
5, POLY(PYRROLE), POLY(STYRENE)
6, POLY(PYRROLE), POLY(A-METHYL STYRENE)
7, POLY(PYRROLE), POLY(STYRENE-ACRYLONITRILE)
8, POLY(PYRROLE), POLY(STYRENE-MALEIC ANYDRIDE)
9, POLY(PYRROLE), POLY(STYRENE-ALLYL ALCOHOL)
10, POLY(PYRROLE), POLY(N-VINYL PYRROLIDONE)
11, POLY(PYRROLE), POLY(4-VINYL PHENOL)
12, POLY(PYRROLE), POLY(VINYL BUTRAL)
13, POLY(PYRROLE), POLY(VINYL ACETATE)
14, POLY(PYRROLE), POLY(BIS PHENOL A CARBONATE)
15, POLY(PYRROLE), POLY(STYRENE)
16, POLY(PYRROLE), POLY(STYRENE)

*FIG. 21A.*

EXPOSE UNKNOWN TO SENSOR ARRAY

INDIVIDUAL SENSOR RESPONSES

SENSOR ARRAY RESPONSE

IDENTIFICATION VIA PATTERN RECOGNITION

SENSOR ARRAY

RESISTANCE
TIME

RESISTANCE
TIME

RESISTANCE
TIME

SENSOR

SUBSTRATE A

SUBSTRATE B

SUBSTRATE C

*FIG. 21B.*

FIG. 21C.

FIG. 22.

FIG. 23A.

FIG. 23B.

INTERDIGITATED ELECTRODES

CERAMIC CAPACITOR

CONDUCTING POLYMER

ELECTRODE CONNECTIONS

FIG. 24A-I.

FIG. 24A-I

SENSOR BUS

FIG. 24A.

SENSOR

%
CHANGE

FIG. 24B.

SENSOR

%
CHANGE

FIG. 24C.

SENSOR

%
CHANGE

FIG. 24D.

SENSOR

FIG. 25.

EP 1 019 715 B1

FIG. 26A.

FIG. 26B.

FIG. 27A.

FIG. 27B.

FIG. 28.

FIG. 29.